(19) Europäisches Patentamt
European Patent Office
Office européen des brevets

(11) EP 2 012 802 B1

(12) FASCICULE DE BREVET EUROPEEN

(45) Date de publication et mention de la délivrance du brevet:
**29.08.2012 Bulletin 2012/35**

(21) Numéro de dépôt: **07731407.8**

(22) Date de dépôt: **03.05.2007**

(51) Int Cl.:
***A61K 31/715*** (2006.01) ***A61K 31/737*** (2006.01)
***A61P 31/22*** (2006.01)

(86) Numéro de dépôt international:
**PCT/FR2007/000761**

(87) Numéro de publication internationale:
**WO 2007/128914 (15.11.2007 Gazette 2007/46)**

# (54) NOUVEAUX MEDICAMENTS POUR LES TRAITEMENTS CONTRE LE VIRUS DE L'HERPÈS

NEUE ARZNEIMITTEL ZUR BEHANDLUNG GEGEN HERPES-VIRUS

NOVEL DRUGS FOR ANTI-HERPES VIRUS TREATMENTS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **04.05.2006 FR 0603990**

(43) Date de publication de la demande:
**14.01.2009 Bulletin 2009/03**

(73) Titulaire: **ASE & BIO**
**75116 Paris (FR)**

(72) Inventeurs:
- **BOURGOUGNON, Nathalie**
  **56890 Saint-Avé (FR)**
- **YVIN, Jean-Claude**
  **35400 Saint Malo (FR)**

(74) Mandataire: **Cabinet Plasseraud**
**52, rue de la Victoire**
**75440 Paris Cedex 09 (FR)**

(56) Documents cités:
**WO-A2-95/24907**

- **BABA M ET AL: "SULFATED POLYSACCHARIDES ARE POTENT AND SELECTIVE INHIBITORS OF VARIOUS ENVELOPED VIRUSES INCLUDING HERPES SIMPLEX VIRUS CYTOMEGALOVIRUS VESICULAR STOMATITIS VIRUS AND HUMAN IMMUNODEFICIENCY VIRUS" ANTIMICROBIAL AGENTS AND CHEMOTHERAPY, vol. 32, no. 11, 1988, pages 1742-1745, XP002413849 ISSN: 0066-4804 cité dans la demande**
- **HASHIMOTO K ET AL: "Antiviral activity of a sulphated polysaccharide extracted from the marine Pseudomonas and marine plant Dinoflagellata against human immunodeficiency viruses and other enveloped viruses" ANTIVIRAL CHEMISTRY AND CHEMOTHERAPY, vol. 7, no. 4, 1996, pages 189-196, XP009076780 ISSN: 0956-3202**
- **KOIZUMI N ET AL: "ANTI-HIV (HUMAN IMMUNODEFICIENCY VIRUS) ACTIVITY OF SULFATED PARAMYLON" ANTIVIRAL RESEARCH, ELSEVIER SCIENCE BV., AMSTERDAM, NL, vol. 1, no. 21, 1993, pages 1-14, XP008003148 ISSN: 0166-3542**
- **KATSURAYA K ET AL: "SYNTHESIS OF SULFATED ALKYL MALTO- AND LAMINARA-OLIGOSACCHARIDES WITH POTENT INHIBITORY EFFECTS ON AIDS VIRUS INFECTION" CARBOHYDRATE RESEARCH, ELSEVIER SCIENTIFIC PUBLISHING COMPANY. AMSTERDAM, NL, vol. 260, no. 1, 1994, pages 51-61, XP008042468 ISSN: 0008-6215**
- **SHIEH M T ET AL: "Cell surface receptors for herpes simplex virus are heparan sulfate proteoglycans." THE JOURNAL OF CELL BIOLOGY. MAR 1992, vol. 116, no. 5, mars 1992 (1992-03), pages 1273-1281, XP002413851 ISSN: 0021-9525**
- **MENARD ROZENN ET AL: "beta-1,3 glucan sulfate, but not beta-1,3 glucan, induces the salicylic acid signaling pathway in tobacco and Arabidopsis" PLANT CELL, vol. 16, no. 11, novembre 2004 (2004-11), pages 3020-3032, XP002453287 ISSN: 1040-4651**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet de nouveaux médicaments pour les traitements contre les herpèsvirus (*Herpèsviridae*).

Il est rappelé que les herpèsvirus appartiennent à une famille de virus dont le génome est à ADN linéaire double brin codant 100 à 200 gènes et qui est encapsulé dans une cage protéinique icosaèdrique appelée la capside, elle-même enveloppée dans une membrane lipidique appelée enveloppe.

La famille des herpèsvirus comporte trois sous familles, à savoir les alpha herpèsvirus, les béta herpèsvirus et les gamma herpèsvirus.

Les alpha herpèsvirus sont caractérisés par un tropisme à l'égard d'un grand nombre de types cellulaires. Parmi les alphavirus, on peut citer le virus Herpès simplex 1 (HSV-1) et le virus Herpès simplex 2 (HSV-2) qui sont responsables de l'herpès buccal et/ou génital, ainsi que le virus varicelle-zona (VZV) responsable de la varicelle et du zona.

Les béta herpèsvirus sont caractérisés par un tropisme à l'égard d'un nombre restreint de types cellulaires. Le cytomégalovirus (CMV) responsable d'un syndrome mononucléosique et les herpès virus humains de type 6 (HHV6) et de type 7 (HHV7) responsables de la roséole, appartiennent à cette sous famille.

Les gamma herpèsvirus sont caractérisés par un tropisme limité aux lymphocytes. Parmi les virus de cette sous famille, on peut citer le virus d'Epstein-Barr (EBV) responsable de la mononucléose infectieuse, du lymphome de Burkitt et du carcinome nasopharyngé, et l'herpèsvirus associé au sarcome de Kaposi ou Rhadinovirus (KSHV) responsable d'un lymphome.

L'infection herpétique comprend une phase d'infection primaire et des phases de latence interrompues par des phases de réactivation. Lors de l'infection primaire ou infection initiale, le virus pénètre dans les cellules épithéliales où il va se multiplier et produire une lyse cellulaire au niveau du site d'inoculation. Le virus peut alors emprunter la voie des nerfs sensitifs pour cheminer jusqu'aux noyaux des neurones des ganglions. Alors que l'infection primaire est souvent accompagnée d'une période courte de maladie clinique, la latence à long terme est asymptomatique. Lors des phases de latence, le virus intracellulaire ne se réplique pas. Le virus demeure dans le noyau des neurones des ganglions nerveux sous forme d'ADN extrachromosomal sans intégrer le génome cellulaire. Le choix des neurones comme site de latence permet aux virus d'échapper au système immunitaire. Suite à certains stimuli tels que les rayonnements ultraviolets, la fièvre ou un stress émotionnel, le virus peut se réactiver et entamer la transcription de nombreux gènes qui conduisent à une réplication accélérée. Cliniquement, la réactivation s'accompagne souvent de l'apparition de symptômes non spécifiques tels qu'une faible fièvre, un état de fatigue, un érythème, ainsi que de signes cliniques tels que des ganglions lymphatiques enflés ou douloureux et de signes immunologiques tels qu'une diminution du nombre des cellules tueuses naturelles.

Une des particularités de l'herpèsvirus réside dans sa capacité à rester présent dans une cellule hôte sous forme latente sans produire de particules virales tout au long de la vie de l'hôte, et dans sa capacité de réactivation, celle-ci pouvant donner lieu à de multiples infections.

La pénétration de l'herpèsvirus dans la cellule hôte constitue une étape essentielle de l'infection. Elle débute par l'adsorption du virus à la surface cellulaire par l'intermédiaire de récepteurs spécifiques et de récepteurs non spécifiques à l'égard des récepteurs comportés par la membrane cellulaire. Après le processus d'adsorption, le virus pénètre dans la cellule par fusion de l'enveloppe virale avec la membrane cellulaire, libérant ainsi la nucléocapside dans le cytoplasme. La nucléocapside est ensuite dégradée par protéolyse enzymatique avec comme conséquence la libération de l'ADN viral qui migre vers le noyau de la cellule hôte et pénètre dans ce dernier.

Une fois parvenu à l'intérieur du noyau, l'ADN viral est transcrit en ARNm viral par l'ARN polymérase II cellulaire. L'expression des gènes viraux est dite "ordonnée", ce qui traduit le fait qu'elle comporte plusieurs phases successives, à savoir une phase dite « très précoce », une phase dite « précoce » et une phase dite « tardive ».

Lors de la phase très précoce, des protéines très précoces virales (Immediate Early Antigens) sont exprimées. Il s'agit de protéines régulatrices qui se fixent sur l'ADN cellulaire et provoquent l'arrêt de la synthèse de certaines protéines cellulaires tout en provoquant l'augmentation de la synthèse d'autres protéines.

Lors de la phase précoce, des protéines enzymatiques virales telles que l'ADN polymérase et la thymidine kinase sont exprimées. Ces deux enzymes sont très importantes pour la réplication du virus. Des mutations de ces enzymes sont responsables de résistance aux agents anti-herpétiques.

Enfin, lors de la phase tardive, ce sont des protéines tardives virales (Late Antigens) qui correspondent entre autres aux protéines de structure de la capside et du tégument qui sont exprimées.

L'assemblage de la nucléocapside des virus nouvellement formés par réplication s'effectue en plusieurs étapes qui demeurent mal définies.

Le virus mature devient infectieux lors de son bourgeonnement au niveau de la membrane nucléaire. Les virus nouvellement formés par réplication sont libérés à l'extérieur de la cellule soit par lyse de la membrane cellulaire, soit par la formation d'une vacuole. La durée du cycle de réplication est d'environ 18 à 20 heures. L'efficacité de la réplication se traduit par le fait qu'il y a synthèse d'une particule virale infectieuse pour 100 à 1000 virus produits dans la cellule hôte.

Les traitements actuellement mis en oeuvre pour combattre les herpèsvirus, notamment HSV-1 et HSV-2, visent à bloquer leur cycle de réplication.

Dans ces traitements, on utilise deux groupes d'agents anti-viraux propres à inhiber la synthèse de l'ADN des herpèsvirus, s'agissant d'une part d'inhibiteurs nucléosidiques et, d'autre part d'inhibiteurs non nucléosidiques de l'ADN polymérase virale.

Parmi les inhibiteurs nucléosidiques de l'ADN polymérase virale, on peut citer l'acyclovir, le penciclovir ainsi que leurs prodrogues respectives, à savoir le valacyclovir et le famcyclovir. Les inhibiteurs nucléosidiques se distinguent des nucléosides naturels par des modifications de leur sucre ou de leur base purique ou pyrimidique. Ils entrent en compétition avec les nucléosides naturels et empêchent l'élongation de la chaîne d'ADN.

L'acyclovir est l'inhibiteur nucléosidique préféré pour le traitement des infections herpétiques. Pour pouvoir agir contre le virus, il doit tout d'abord être phosphorylé par l'enzyme virale thymidine kinase (TK) ; il doit ensuite être soumis à d'autres phosphorylations réalisées par des enzymes cellulaires ce qui permet d'aboutir à sa forme active. Sous cette forme active, l'acyclovir est un inhibiteur très sélectif de l'ADN polymérase virale qu'il inhibe davantage que l'ADN polymérase cellulaire.

Pour ce qui est des inhibiteurs non nucléosidiques, ce sont des analogues de pyrophosphate anorganique. Ces molécules n'ont pas à être préalablement phosphorylées pour inhiber les enzymes virales. Les traitements à base d'inhibiteurs non nucléosidiques sont utilisés dans le cas d'infections résistantes aux analogues nucléosidiques. Parmi les inhibiteurs non nucléosidiques de l'ADN polymérase virale on peut citer le Foscarnet.

Actuellement, les limites des traitements utilisés pour combattre les herpèsvirus, notamment HSV-1 et HSV-2 sont dues à la toxicité des inhibiteurs mis en oeuvre, à l'émergence de souches virales résistantes ainsi qu'au fait que ces traitements ne sont actifs que sur des virus en phase de réplication.

Le mécanisme de résistance de certaines souches virales à l'acyclovir est principalement dû à la présence de mutations sur le gène codant pour la TK virale, ce qui induit une altération de sa fonction. Ainsi, une TK déficiente ne sera plus en mesure de phosphoryler l'acyclovir. Mais une souche virale mutante comportant une TK déficiente n'en reste pas moins viable car l'enzyme TK n'est pas essentielle pour la réplication du virus.

Chez les individus immunocompétents, l'apparition de souches HSV résistantes à l'acyclovir est très rare mais a été signalée. En revanche, ce risque est plus grand dans le cas des individus immunodéficients, c'est-à-dire ceux qui sont soumis à une thérapie immunodépressive ce qui est le cas notamment pour les personnes ayant subi une transplantation d'organe, ceux atteints du syndrome de l'immunodéfience acquise (SIDA) ou ceux qui présente des grandes altérations de la peau tels que les grands brûlés.

Plusieurs facteurs seraient associés à ce phénomène de résistance rencontré chez les individus immunodéficients. Il y a tout d'abord l'hétérogénéité des populations virales présentes chez un individu infecté. Certaines souches mutantes seraient issues de sous-populations virales minoritaires naturellement résistantes à un antiviral donné (ainsi, environ 0,01% des virus HSV sont spontanément résistants à l'acyclovir ou ACV). Il y a également le fait que le degré d'immunodéficience d'un individu infecté semblerait jouer un rôle important en influençant les facteurs immunitaires qui interviennent dans la limitation de la réplication virale. Enfin, l'usage prolongé d'agents antiviraux à des doses parfois suboptimales peut favoriser le développement de la résistance virale (Englund et al., Ann. Intern. Med., 1990, 112 : 416-22).

Comme déjà indiqué plus haut, le Foscarnet est actuellement le seul agent anti-herpèsvirus approuvé pour le traitement des infections causées par des HSV résistants aux analogues nucléosidiques. Cependant, un traitement à long terme au Foscamet entraîne fréquemment une forte intolérance.

Enfin, il n'existe pas, actuellement, de vaccin anti-Herpès permettant un traitement de prévention.

Au vu des considérations qui précèdent, il est devenu nécessaire de faire porter l'effort de recherche sur d'autres cibles thérapeutiques et de mettre au point de nouveaux agents antiviraux permettant de combattre l'infection herpétique.

Il s'est avéré qu'une telle cible thérapeutique prometteuse se situe au niveau de l'entrée des particules virales dans la cellule, et plus particulièrement au niveau du mécanisme d'adsorption de ces particules virales à la surface cellulaire ; un intérêt particulier de cette cible est qu'elle se situe en amont de la pénétration des nucléocapsides dans les cellules, autrement dit de la réplication virale.

L'adsorption est une étape critique de l'infection.

Il est déjà connu que certains polysaccharides sulfatés dont notamment le sulfate de dextran étaient capables d'interférer dans le mécanisme d'adsorption de certains virus enveloppés, s'agissant notamment du HSV-1 et du HSV-2. (Baba et al., Antimicrobial Agents and Chemotherapy, 1988, 32 : 1742-1745).

Ainsi, Mazumder et al. (Inter. J. Biol. Macromol, 2002, 31 : 87-95) ont montré qu'un polysaccharide de très haut poids moléculaire (165197 Da), extrait d'une algue rouge *Gracilaria corticata* exerçait un effet d'inhibition à l'égard de l'adsorption de virus HSV-1 et HSV-2 sur des cellules Vero.

Par ailleurs, Yingzhou et al. (China J.I., 2004, 6 : 23) ont montré qu'un polysaccharide extrait d'une autre espèce d'algue rouge *Eucheuma striatum* avait également un effet inhibiteur sur l'adsorption du virus HSV-1 sur des cellules

*in vitro.*

BABA M ET AL: "SULFATED POLYSACCHARIDES ARE POTENT AND SELECTIVE INHIBITORS OF VARIOUS ENVELOPED VIRUSES INCLUDING HERPES SIMPLEX VIRUS CYTOMEGALOVIRUS VESICULAR STOMATITIS VIRUS AND HUMAN IMMUNODEFICIENCY VIRUS" ANTIMICROBIAL AGENTS AND CHEMOTHERAPY, vol. 32, no. 11, 1988, pages 1742-1745, décrit l'activité d'un nombre de polysaccharides sulfatés, entre autre du dextran sulfate et de l'héparine, contre les virus (soit DNA - soit RNA -) enveloppés et en particulier contre le HSV -1 et le HSV -2. L'activité est attribuée à l'inhibition de l'absorption virale.

Ces travaux n'ont toutefois pas conduit à la mise au point de nouveaux traitements contre les herpèsvirus.

L'invention a donc pour but, surtout, de mettre à la disposition du corps médical de nouveaux médicaments à indice thérapeutique élevé pour lutter contre les herpèsvirus en général, et plus particulièrement contre les alpha herpèsvirus, et notamment contre les HSV-1 et HSV-2, ces médicaments s'opposant essentiellement à la multiplication desdits virus par inhibition de l'adsorption de ces derniers sur la cellule hôte.

Et il est du mérite de la Société Demanderesse d'avoir trouvé que, de façon surprenante et inattendue, ce but pouvait être atteint par l'utilisation de polysaccharides particuliers.

La présente invention a donc pour premier objet l'utilisation, pour la préparation d'un médicament destiné au traitement des maladies causées par les herpèsvirus, plus particulièrement par les alpha herpèsvirus, et notamment par les HSV-1 et HSV-2, ainsi que par des souches de ces virus qui sont résistantes aux agents antiviraux déjà connus, d'au moins un polysaccharide de formule (I)

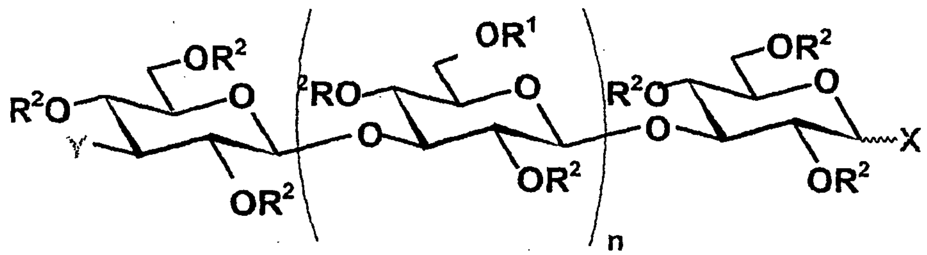

**(I)**

dans laquelle - $R^1$ représente soit un atome d'hydrogène, un groupement sulfate soit un glucose sulfaté lié, de préférence, par une liaison de type $\beta(1\rightarrow6)$ à la structure saccharidique,

- $R^2$ représente un atome d'hydrogène, un groupement sulfate $R^1$ et $R^2$ ne pouvant pas représenter simultanément un atome d'hydrogène,
- X et Y représentent, chacun indépendamment, un groupement OH, un glucose, un glucose sulfaté ou phosphaté, un mannitol, ou un mannitol sulfaté,
- n représente un nombre entier de 11 à 30, préférentiellement de 20 à 30, plus préférentiellement de 25 à 30,

ledit polysaccharide ayant un degré de sulfatation supérieur à 2, préférentiellement de 2,2 à 2,4, ledit médicament agissant notamment par inhibition de l'adsorption des virus en question sur la cellule hôte.

Dans un mode de réalisation particulier, le polysaccharide utilisé est un polysaccharide de formule (I), dans laquelle $R_1$ et $R_2$ peuvent être soit identiques et représentent alors un groupement sulfate soit différents l'un de l'autre, $R_1$ représentant alors une unité glucose sulfatée liée, de préférence, par une liaison β de type β-1,6 à la structure saccharidique, X et/ou Y représentant un groupement mannitol et n un nombre entier de 11 à 30, plus particulièrement de 25 à 30, ledit médicament agissant notamment par inhibition de l'adsorption des virus en question sur la cellule hôte.

De manière étonnante et inattendue, la Demanderesse a par ailleurs démontré que le médicament fabriqué par utilisation d'un polysaccharide de formule (I) conformément à l'invention, agit par inhibition de l'adsorption des virus en question sur la cellule hôte. De plus, la Demanderesse a pu démontré également que les polyscchamides de formule (I) selon l'invention, et en particulier la laminarine sulfatée, ne présentaient ni de toxicité *in vitro* et *in vivo,* ni d'activité anti-anticoagulante et donc pas de risques d'effets secondaires hémorragiques, et présentaient un indice thérapeutique élevé.

Par « indice thérapeutique » ou « IT », au sens de l'invention, on entend le rapport entre la dose maximale non toxique (ou une dose induisant une toxicité quantifiée) et la dose induisant un effet pharmacologique favorable.

Au sens de la présente invention, on entend par « degré de sulfatation », le nombre moyen, par unité saccharidique, de groupements OH sulfatés. Un degré de sulfatation supérieur à 2 signifie que, en moyenne, sur l'ensemble du polysaccharide, plus de 2 groupements OH par unité saccharidique sont sulfatés.

Au sens de l'invention, on entend par « groupement sulfate », un groupement du type ($-SO_3H$).

Un autre objet de la présente invention est l'utilisation, pour la mise en oeuvre d'une méthode de traitement de maladies causées par les herpèsvirus, plus particulièrement par les alpha herpèsvirus, et notamment par les HSV-1 et HSV-2, ainsi que par des souches de ces virus qui sont résistantes aux agents antiviraux déjà connus, d'au moins un polysaccharide de formule (I) telle que définie précédemment.

Selon un mode de réalisation avantageux, ledit polysaccharide de formule (I) est une laminarine sulfatée ayant un degré de sulfatation supérieur à 2 et de préférence de 2,2 à 2,4. De préférence, la laminarine sulfatée utilisée selon l'invention présente un degré de polymérisation de 11 à 28.

Préférentiellement, le polysaccharide de formule (I) est une laminarine sulfatée de degré de sulfatation égal à environ 2,3, et de degré de polymérisation de 11 à 28, appelée « laminarine PS3 ».

Par « degré de polymérisation » on entend, au sens de l'invention, le nombre d'unités monosaccharidiques liées entre elles par des liaisons de type $\beta(1\rightarrow3)$ composant la chaîne linéaire principale. Un degré de polymérisation de 11 à 28 signifie un polysaccharide composé de 11 à 28 unités saccharidiques, notamment glucose, liées entre elles par des liaisons de type $\beta(1\rightarrow3)$. Ce degré de polymérisation ne tient pas compte des unités glucoses liées en $\beta(1\rightarrow6)$ à la chaine principale du polysaccharide. Ainsi, le degré de polymérisation est égal à n+2 lorsque X et Y représentent simultanément OH, à n+3 si seulement l'un de X ou Y représente OH, et à n+4 si ni X ni Y ne représente OH.

De manière étonnante et surprenante, la Demanderesse a pu démontrer que la laminarine sulfatée, ayant un degré de sulfatation supérieur à 2, de préférence de 2,2 à 2,4, et un degré de polymérisation de 11 à 28, était particulièrement efficace pour le traitement des maladies causées par les herpèsvirus, de préférence choisies parmi celles causées par les alpha herpèsvirus, et notamment par les HSV-1 et HSV-2, ainsi que par des souches de ces virus qui sont résistantes aux agents antiviraux déjà connus. Cette laminarine sulfatée présente en outre une faible activité anticoagulante, sans risques d'effets secondaires hémorragiques, confirmant ainsi son grand intérêt pour la fabrication d'un médicament destiné à une administration humaine ou animale, et présente également un indice thérapeutique élevé.

Selon un autre mode de réalisation avantageux, l'invention vise l'utilisation d'un oligosaccharide, obtenu à partir de la laminarine sulfatée de degré de sulfatation supérieur à 2 et de préférence de 2,2 à 2,4, le degré de polymérisation de cet oligosaccharide étant de 11 à 28, pour la préparation d'un médicament pour les traitements contre les herpèsvirus en général, plus particulièrement contre les alpha herpèsvirus, et notamment contre les HSV-1 et HSV-2, ce médicament qui est également actif contre des souches résistantes aux agents antiviraux déjà connus agissant sur la multiplication desdits virus notamment par inhibition de l'adsorption de ces derniers sur la cellule hôte.

Un mode de réalisation particulier de l'invention concerne l'utilisation d'une laminarine sulfatée, caractérisée en ce qu'elle possède un degré de sulfatation supérieur à 2, de préférence de 2,2 à 2,4, et un degré de polymérisation de 11 à 28, pour la fabrication d'un médicament destiné au traitement des maladies causées par les herpèsvirus.

Un autre mode de réalisation particulier de l'invention concerne l'utilisation d'une laminarine sulfatée, caractérisée en ce qu'elle possède un degré de sulfatation supérieur à 2, de préférence de 2,2 à 2,4, et un degré de polymérisation de 11 à 28, pour la mise en oeuvre d'une méthode de traitement des maladies causées par les herpèsvirus.

Selon un mode de réalisation préféré de l'invention, les maladies causées par les herpèsvirus sont préférentiellement choisies parmi celles des individus immunodéficients, plus particulièrement parmi celles des individus transplantés, des individus atteints du syndrome de l'immunodéfience acquise ou des individus qui présentent des grandes altérations de la peau, et notamment des grands brûlés.

En particulier, on entend par « maladie causée par les herpèvirus » au sens de l'invention, notamment l'herpès buccal, l'herpès génital, la varicelle, le zona, la mononucléose infectieuse, le lymphome de Burkitt, le carcinome nasopharyngé, le syndrome mononucléosique, la rétinite, la roséole, le sarcome de Kaopsi, ou encore tous les lymphomes associés aux herpèsvirus.

De plus, on entend par « herpèsvirus » ou *« herpesviridae »*, au sens de l'invention, les virus herpétiques humains et animaux, appartenant notamment aux familles des alphaherpesvirinae, betaherpesvirinae, et des gammaherpevirinae. Plus particulièrement, l'invention concerne les virus du type simplexvirus, varicellovirus, mardivirus, iltovirus, cytomegalovirus, muromegalovirus, roseolovirus, lymphocryptovirus, rhadinovirus, ou encore les ictaluvirus. Préférentiellement, on entend par herpèsvirus au sens de l'invention les virus HSV-1, HSV-2, VZV (varicella zoster virus), HHV-4 ou EBV (Epstein Barr virus), HHV-5 ou CMV, HHV-7, et HHV-8 ou KSHV.

Selon un mode de réalisation avantageux, l'invention vise l'utilisation d'un polysaccharide de formule I, plus particulièrement de la laminarine sulfatée ayant un degré de sulfatation supérieur à 2 et de préférence de 2,2 à 2,4 pour la préparation d'un médicament pour les traitements contre les alpha herpèsvirus, et notamment contre les HSV-1 et HSV-2, tout particulièrement destiné aux individus immunodéficients, plus particulièrement à des individus transplantés, des individus atteints du syndrome de l'immunodéfience acquise (SIDA) ou des individus qui présentent des grandes altérations de la peau, et notamment les grands brûlés.

L'invention a également pour objet une composition pharmaceutique comprenant au titre de substance active une quantité efficace d'au moins un polysaccharide de formule 1 telle que définie précédemment, et plus particulièrement de laminarine sulfatée ayant un degré de sulfatation supérieur à 2 et de préférence de 2,2 à 2,4, ainsi qu'une quantité efficace d'au moins un composé choisi parmi le groupe comprenant les inhibiteurs nucléosidiques et les inhibiteurs non-nucléosidiques d'une enzyme virale d'un herpèsvirus.

Au sens de l'invention, on entend par « inhibiteurs nucléosidiques », notamment et sans s'y limiter l'acyclovir, le penciclovir, le cidofovir, le ganciclovir, le valganciclovir, le valacyclovir et le famcyclovir.

Au sens de l'invention, on entend par « inhibiteurs non nucléosidiques », notamment les analogues de pyrophosphate anorganique, en particulier le foscarnet.

Dans une telle composition pharmaceutique, le polysaccharide de formule (I) et le composé choisi parmi le groupe comprenant les inhibiteurs nucléosidiques et les inhibiteurs non-nucléosidiques d'une enzyme virale d'un herpèsvirus, peuvent être utilisés de manière simultanée, séparée ou étalée dans le temps.

En effet, l'homme du métier est à même de définir l'administration la mieux adaptée permettant d'obtenir le meilleur indice thérapeutique pour le patient. Chaque substance active peut être administrée de manière séquentielle, voire par des routes différentes, ou alors en même temps.

Par « quantité efficace », au sens de l'invention, on entend une quantité de substance active suffisante pour obtenir un effet thérapeutique sur un patient.

L'invention concerne aussi une méthode de traitement de maladies causées par les herpèsvirus, plus particulièrement par les alpha herpèsvirus, et notamment par les HSV-1 et HSV-2, ainsi que par des souches de ces virus qui sont résistantes aux agents antiviraux déjà connus, comprenant l'administration, chez un patient atteint par ladite maladie causée par les herpèsvirus, d'une quantité efficace d'un médicament comprenant à titre d'agent actif au moins un polysaccharide de formule (I) telle définie précédemment.

Au sens de la présente invention, par « patient » on entend tout animal à sang chaud, particulièrement les mammifères et notamment les êtres humains.

Dans un mode de réalisation particulier, le polysaccharide de formule (I) mis en oeuvre dans la méthode de traitement selon l'invention est une laminarine sulfatée, caractérisée en ce qu'elle possède un degré de sulfatation supérieur à 2, de préférence de 2,2 à 2,4, et un degré de polymérisation de 11 à 28.

Dans un mode réalisation avantageux, la méthode de traitement telle que définie précédemment, comprend, en plus de l'administration d'au moins un polysaccharide de formule (I) telle définie précédemment, l'administration simultanée ou séquentielle d'une quantité efficace d'au moins un composé choisi dans le groupe comprenant les inhibiteurs nucléosidiques et les inhibiteurs non-nucléosidiques d'une enzyme virale d'un herpèsvirus.

Dans un mode de réalisation particulier, la méthode de traitement selon l'invention est caractérisée en ce que lesdits patients atteints par une maladie causée par les herpèsvirus sont choisis parmi les individus immunodéficients, plus particulièrement parmi les individus transplantés, les individus atteints du syndrome de l'immunodéficience acquise ou les individus qui présentent de grandes altérations de la peau, et notamment les grands brûlés.

Pour préparer un polysaccharide de formule (I) sulfaté au sens de l'invention, on effectue une étape de sulfatation en suivant de préférence le protocole décrit par Alban S, Kraus J, et Franz G dans « Synthesis of laminarin sulfates with anticoagulant activity », Artzneim.Forsch./drug Res (1992) 42 ; 1005-1008. Ce procédé a été perfectionné dans la thèse de Susanne Alban, soutenue en 1993 à l'Université de Regensburg et portant le titre « Synthese und physiologische Testung neuartiger Heparinoide ». Ces procédés sont adaptables à la sulfatation des polysaccharides de formule (I) de l'invention et permettent d'obtenir un polysaccharide sulfaté hautement substitué, sans dégradation, et avec une bonne reproductibilité, d'une manière simple et peu onéreuse.

Pour aboutir à une sulfatation efficace du polysaccharide sans dégradation des chaînes polysaccharidiques, la réaction de sulfatation est avantageusement effectuée sous des conditions correspondant à une absence absolue d'eau. Avant la sulfatation, le polysaccharide est donc de préférence séché, par exemple sur pentoxide de phosphore ($P_2O_5$) et ensuite dissout dans du diméthylformamide ou DMF. De par ses effets alternatifs sur le polysaccharide, le DMF a une influence activante par la substitution. En effet, l'association du DMF polaire avec les groupes OH conduit à la coupure des liaisons hydrogène intra et inter moléculaires et à la désintégration des structures supérieures.

Pour mettre en oeuvre la réaction de sulfatation, on peut avoir recours avantageusement au complexe $SO_3$-pyridine.

Par suite de la coordination de l'accepteur d'électrons $SO_3$ avec le donneur d'électrons pyridine, la réactivité difficilement contrôlable du $SO_3$ qui se traduit par des réactions fortement exothermiques entraînant des dégradations, se trouve réduite. Le complexe $SO_3$-pyridine présente par rapport à d'autres complexes l'avantage d'être ni trop réactif ni trop stable c'est-à-dire trop lent du point de vue réaction.

En raison du fait que le degré de sulfatation obtenu est proportionnel à l'excès molaire en réactif de sulfatation et étant donné que l'on cherche à obtenir un degré de substitution supérieur à 2, on met avantageusement en oeuvre une concentration de 6 moles de $SO_3$-pyridine par mole de glucose.

Avantageusement, pour garantir l'absence d'eau, on peut travailler sous atmosphère d'argon.

De préférence, on ajoute dès le début de la réaction de la pyridine au réactif de sulfatation et ce, en quantité équimolaire, en vue de capter directement l'acide sulfurique qui pourrait se former par réaction du complexe $SO_3$-pyridine avec l'eau. La concentration du polysaccharide ainsi que celle du réactif de sulfatation doivent être de préférence aussi élevées que possible, la solubilité du polysaccharide et du réactif de sulfatation limitant le degré de sulfatation final.

Pour éviter au début de la réaction un refroidissement du mélange qui pourrait entraîner des problèmes de solubilité et pour obtenir une substitution la plus régulière possible, la solution du complexe $SO_3$-pyridine dans le DMF pourrait ne pas être ajoutée en une seule fois mais de manière continue pendant une durée de 4 heures.

La réaction de sulfatation peut être effectuée à une température de 20 à 60°C, de préférence d'environ 40°C. Des températures plus élevées entraînent une substitution plus efficace mais, également, une dégradation des chaînes.

Après l'addition du réactif de sulfatation, le mélange est de préférence agité pendant plusieurs heures autour de 60°C. A cette température, il se produit une substitution supplémentaire sans dégradation des chaînes.

Le surnageant du mélange est alors avantageusement séparé par décantation. Le résidu est dissous, de préférence dans du NaOH, puis mélangé avec 10 fois son volume d'éthanol. Le précipité qui se produit à une température de 4-8°C pendant la nuit est isolé puis préférentiellement dissous dans de la soude diluée (solution de pH d'environ 9). La solution est dialysée pour enlever les sels et les molécules de bas poids moléculaire puis avantageusement amenée à un pH de 7,0 par addition de NaOH et ensuite lyophilisée. Le polysaccharide sulfaté résultant se présente sous forme de sel de sodium.

Le degré de sulfatation est préférentiellement déterminé par voie de titration conductimétrique de l'acide libre du polysaccharide sulfaté, ou alternativement par chromatographie ionique après hydrolyse en utilisant un système du type HPLC. La première méthode présente l'avantage d'être également propre à des recherches relatives à la stabilité (la consommation de soude s'accroît lorsque des groupes sulfates sont éliminés) alors que la méthode HPLC nécessite moins de substance et peut être automatisée. A titre de contrôle, il est possible de déterminer la teneur en soufre par analyse élémentaire.

Il est de plus possible de contrôler l'homogénéité de la sulfatation et la répartition des groupes sulfate sur les différentes positions dans la molécule de glucose par une forme modifiée de l'analyse de méthylation suivie d'un examen GC-MS (à savoir Chromatographie Gaz, Spectrométrie de Masse).

Le degré de sulfatation obtenu en procédant comme indiqué ci-dessus est supérieur à 2, plus précisément de 2 à 2,5 et tout particulièrement de 2,2 à 2,4.

Suivant un mode de réalisation avantageux, le polysaccharide de formule (I), et, de préférence, la laminarine sulfatée, sont utilisés pour la préparation d'un médicament pour le traitement des maladies causées par les herpèsvirus destiné à une administration par voie générale et de préférence par les voies orale, rectale, pulmonaire topique (incluant les voies transdermique, buccale et sublinguale) et parentérale (incluant les voies sous-cutanées intramusculaire, intraveineuse, intradermique et intra-vitréale).

La dose quotidienne est généralement de 0,01 à 250 mg par kilo de poids du patient et préférentiellement de 0,10 à 100 mg, plus préférentiellement encore de 0,5 à 30 mg, et tout particulièrement de 1,0 à 20 mg.

Ces doses quotidiennes s'appliquent notamment dans le cas de la laminarine sulfatée ; pour les autres sels et esters selon la formule (I), les doses quotidiennes sont adaptées à chaque cas.

La dose quotidienne peut être administrée par dose unitaire en une, deux, trois, quatre, cinq ou six fois ou plus à différents moments de la journée.

Les doses unitaires peuvent comporter de 10 à 1000 mg, de 50 à 400 mg, et, préférentiellement, de 50 à 100 mg de substance active.

Les médicaments obtenus, conformément à l'invention, en utilisant au moins un des polysaccharides de formule (I), comportent les ingrédients de formulation classiques et éventuellement un ou plusieurs autres agents thérapeutiques.

De plus, comme mentionné précédemment, le polysaccharide de l'invention peut être avantageusement combiné avec d'autres substances actives. Leur mode d'administration peut être simultané ou séquentiel. Elles peuvent également être administrées par différentes voies telles que décrites précédemment.

La présente invention sera mieux comprise à la lecture des exemples non limitatifs suivants.

La figure 1 montre la courbe de titrage d'une suspension stock d'une souche HSV-1 : les pourcentages de cellules Vero détruites sont exprimés en fonction des dilutions virales de la suspension stock (de $10^{-1}$ à $10^{-8}$).

La figure 2 montre l'évolution de la valeur $CE_{50}$ du PS3 ($\mu$g/ml) dans le cas de cellules Vero infectées par des suspensions virales HSV-1 ayant des MOI différentes (MOI 0,01 $ID_{50}$/cellule, courbe A, MOI 0,1 $ID_{50}$/cellule, courbe B et MOI 1 $ID_{50}$/cellule, courbe C), en fonction des temps d'incubation du PS3 (exprimé en heures h).

La figure 3 est un diagramme montrant les valeurs $CE_{50}$ du PS3 et de l'acyclovir pour chacune des souches virales étudiées, HSV-1, HSV-IR, HSV-2 et HSV-2R, après un traitement de 48 heures des cellules Vero infectées.

La figure 4 est un diagramme montrant les valeurs $CE_{50}$ du PS3 et de l'acyclovir pour chacune des souches virales étudiées HSV-1, HSV-IR, HSV-2 et HSV-2R, après un traitement de 72 heures des cellules Vero infectées.

La figure 5 est un graphique montrant une courbe D traduisant l'activité cytotoxique du PS3, les pourcentages de destruction cellulaires ($CC_{50}$) étant exprimés en fonction des temps d'incubation du PS3 (heures h).

La figure 6 est un diagramme montrant les valeurs $CE_{50}$ du PS3 et de l'acyclovir obtenues pour des cellules Vero infectées par les suspensions virales HSV-1, HSV-1R, HSV-2 et HSV-2R préalablement traitées par le PS3 ou l'acyclovir, après 48 heures de culture.

La figure 7 est un diagramme montrant les valeurs $CE_{50}$ du PS3 et de l'acyclovir obtenues pour des cellules Vero infectées par les suspensions virales HSV-1, HSV-1R, HSV-2 et HSV-2R préalablement traitées par le PS3 ou l'acyclovir, après 72 heures de culture.

La figure 8 regroupe les quatre graphiques 8A, 8B, 8C et 8D sur lesquels apparaissent les courbes représentant l'évolution des valeurs $CE_{50}$ du PS3 et de l'acyclovir en fonction de la durée de contact du PS3 ou de l'acyclovir avec les cellules Vero infectées par les souches virales HSV-1 (graphique 8A), HSV-1R (graphique 8B) ; HSV-2 (graphique 8C) et HSV-2R (graphique 8D), respectivement.

La figure 9 regroupe les quatre graphiques 9A, 9B, 9C et 9D qui mettent en évidence l'effet du PS3 sur l'adsorption virale des souches virales HSV-1, HSV-1R, HSV-2 et HSV-2R respectivement par mesure des valeurs $CE_{50}$ du PS3 et de l'acyclovir déterminées sur des cellules Vero traitées pendant l'infection (traitement A), après l'infection (traitement B), ou pendant et après l'infection (traitement C).

**Exemple 1 : Préparation d'un sulfate de laminarine PS3**

En premier lieu, on a extrait la laminarine d'une matière première constituée par des algues brunes en suivant le protocole décrit dans le brevet FR 92 08387.

Une fois la laminarine extraite, on a effectuée une sulfatation en suivant le protocole décrit par Alban S, Kraus J, et Franz G dans Synthesis of laminarin sulfates with anticoagulant activity, Artzneim.Forsch./drug Res (1992) 42 ; 1005-1008, perfectionné par la thèse de Susanne Alban, soutenue en 1993 à l'Université de Regensburg et portant le titre « Synthese und physiologische Testung neuartiger Heparinoide ».

On a d'abord séché la laminarine sur du pentoxide de phosphore ($P_2O_5$) puis on l'a dissoute dans du diméthylformamide ou DMF.

Ensuite, pour mettre en oeuvre la réaction de sulfatation, on a eu recours au complexe $SO_3$-pyridine sous atmosphère d'argon : on a ajouté en une seule fois mais de manière continue pendant une durée de 4 heures de la $SO_3$-pyridine dans le DMF en quantité équimolaire. On a réalisé la réaction de sulfatation à une température de 40°C. Après l'addition du réactif de sulfatation, on a continué à agiter le mélange pendant 6 heures à 60°C.

On a ensuite séparé le surnageant du mélange par décantation, dissous le résidu dans 2,5 M de NaOH, puis on l'a mélangé avec 10 fois son volume d'éthanol à 99%. On a alors placé la solution obtenue à une température de 4-8°C pendant la nuit et on a obtenu un précipité que l'on a isolé puis dissous dans de la soude diluée (solution de pH d'environ 9). Ensuite on a dialysé la solution à l'aide d'une membrane Spectrapor à seuil de coupure 1000 D puis amené le pH à 7,0 par addition de NaOH. Enfin, on a lyophilisé la solution ainsi dialysée. On a obtenu un sulfate de laminarine se présentant sous forme de sel de sodium.

On a ensuite déterminé le degré de sulfatation par voie de titration conductimétrique de l'acide libre du polysaccharide sulfaté en utilisant de la soude 0,1N. Le degré de sulfatation de la laminarine obtenu était de 2,3. Le degré de polymérisation du sulfate de laminarine ainsi obtenu était de 23 à 28.

On a nommé ce polysacharide sulfaté « laminarine sulfatée PS3 », ou encore « PS3 ».

**Exemple 2 : Préparation des cellules et des souches virales**

Pour la mise en oeuvre des différents tests décrits ci après, on a utilisé des cellules Vero, qui sont des cellules fibroblastiques de rein de singe vert (Cercopithecus aethiops, ATCC : CCL 81). Ces cellules ont un taux de croissance de 1 :20 en 7 jours quand elles sont ensemencées à une concentration de $3x10^5$ cellules/ml dans un milieu de culture MEM supplémenté en sérum de veau foetal.

On a de plus mis en oeuvre les souches virales suivantes :

- la souche 17 sauvage de HSV-1 ACVs et PFAs
- la souche HSV-2 ,
- la souche HSV-1R résistante à l'Acyclovir,
- la souche HSV-2R résistante à l'Acyclovir.

Ces souches virales ont été fournies par le Professeur Ingrand (Laboratoire de virologie de Reims, France). Pour obtenir un stock viral pour chacune de ces souches, on a procédé de la façon suivante.

On a cultivé des cellules Vero dans un flacon jusqu'à confluence (350 000 cellules/ml) dans 5 ml de milieu minimum essentiel de Eagle «Minimum Essential Medium Eagle» (MEM) additionné de 8% de sérum de veau foetal (SVF) contenant 500 μl de suspension virale.

On a ensuite placé le flacon de culture dans une étuve à 37°C pendant 2h. Après cette incubation, on a rincé les cellules puis on les a recouvertes de milieu MEM contenant 8% de SVF.

Après 3 à 4 cycles de multiplication virale correspondant à environ 3 jours de culture, on a soumis le flacon à

2 cycles successifs de congélation et de décongélation afin de faire éclater les cellules et libérer les virions intracellulaires.

On a alors récupéré le surnageant de culture puis on l'a centrifugé 10 minutes à 600g à l'aide d'une centrifugeuse Jouan MR22i, de façon à éliminer les débris cellulaires.

On a ensuite congélé le surnageant de culture à -80°C qui constituait le stock viral. On a alors titré ce stock viral, puis on l'a réparti dans des cryotubes par aliquots de 2 ml et on les a conservés à -80°C.

Pour chaque souche virale, on a déterminé le titre infectieux qui correspond au nombre de particules virales par unité de volume capables d'infecter des cellules permissives.

On a réalisé ce titrage selon le protocole suivant dans des plaques de microtitration de 96 puits. Chaque plaque de microtitration se compose de 8 lignes de 12 puits formant par conséquent 12 colonnes de 8 puits. Chaque ligne correspond à une dilution de la suspension virale stock de $10^{-1}$ à $10^{-8}$.

On a ensemencé des cellules Vero à une densité cellulaire de $3{,}5.10^4$ cellules/puits dans 100 µl de milieu MEM contenant 8% de SVF :

- on a complété une des colonnes, dite colonne témoin négatif, avec 50 µL de milieu MEM avec 8% de SVF,
- on en a complété une autre, dite colonne témoin positif, avec 50 µL de surnageant de culture issu du stock viral,
- enfin on a successivement complété les dix autres colonnes avec 50 µL de surnageant viral dilué de 10 en 10 dans du milieu MEM contenant 8% de SVF.

On a ensuite placé la plaque de microtitration dans une étuve à 37°C pendant 72 heures. Pendant cette période, quatre à cinq cycles de multiplication du virus se produisent. Après 72 heures, on a éliminé le surnageant et on a examiné l'effet cytopathique du virus HSV. On a visualisé cet effet cytopathique d'une part par un gonflement et un arrondissement des noyaux des cellules qui prenaient un aspect globuleux et d'autre part, par une organisation des cellules infectées en chapelets.

On a alors déterminé le titre de chaque souche virale par la méthode de Reed et Muench (Am, J. Hyg., 1983, 27 : 493-497). Selon cette méthode de titrage, on a compté, pour chaque dilution virale, le nombre de puits présentant un effet cytopatique ainsi que le nombre de puits ne présentant pas d'effet cytopatique. On a choisi la gamme de dilutions employée suffisamment large pour inclure d'une part la dilution pour laquelle toutes les réponses sont positives et, d'autre part, la dilution pour laquelle toutes les réponses sont négatives.

On a alors exprimé le titre infectieux en $DI_{50}$/ml, qui désigne la dose qui infecte 50% des cellules de la culture considérée. On a calculé ce titre selon une formule statistique, après avoir déterminé les dilutions encadrant le point d'infection 50% :

$$\text{Log } DI_{50} = \text{Log}10^{-(\text{dilution supérieure à } 50\%+d)} + d$$

$$\text{où} \quad d = \frac{(\%\text{ dilution supérieure à } 50\% - 50\%)}{(\%\text{ dilution supérieure à } 50\% - \%\text{ dilution inférieure à } 50\%)}$$

A partir de ce calcul, on a déterminé ce qu'on appelle la multiplicité d'infection MOI (Multiplicity Of Infection) en fonction du nombre de cellules.

A titre d'exemple, la dose infectieuse à 50% de la souche virale HSV-1 est de $10^{4,75}$ $DI_{50}$ pour 50 µl soit un titrage de $2x10^{5,75}$ $DI_{50}$ pour 1 ml selon la méthode de Reed et Muench.

Parallèlement à cette méthode, on a également déterminé le titre infectieux de la suspension stock de chaque souche virale par la mesure par coloration au rouge neutre de la viabilité de cellules infectées.

Cette méthode repose sur le principe que les cellules vivantes absorbent le rouge neutre qui est un colorant vital. On a mesuré la densité optique du milieu à la longueur d'onde d'absorption maximale du rouge neutre, c'est-à-dire 540 nm, à l'aide d'un spectrophotomètre (SpectraCount TM, microplate photometer, Packard). La densité optique à 540 nm est proportionnelle au nombre de cellules vivantes dans une culture infectée (McLaren et al, Antiviral Research, 1983 ; 3 : 223-234 et Langlois et al, Standardization, 1986, 14 : 201-211).

On a reporté la courbe de titrage de la souche virale HSV-1 obtenue par la méthode de viabilité cellulaire au rouge neutre à la figure 1. La dose infectieuse à 50% de la souche virale HSV-1 est de $10^{5,05}$ pour 50 µl ce qui correspond à un titre infectieux de $2x10^{6,05}$ pour 1 ml ; la valeur de $2x10^{6,05}$ est la moyenne de 4 titrages réalisés pour la souche HSV-1.

Le même protocole a permis de déterminer le titre viral de chaque suspension stock :

$2x10^{6,05}$ $DI_{50}$/ml pour HSV-1

$2x10^{4,42}$ $DI_{50}$/ml pour HSV-1R
$2x10^{5,31}$ $DI_{50}$/ml pour HSV-2
$2x10^{4,44}$ $DI_{50}$/ml pour HSV-2R

On a alors souhaité mettre en évidence l'activité antivirale de la laminarine sulfatée PS3 sur plusieurs souches virales par appréciation de la viabilité de cellules Vero infectées en présence de la laminarine sulfatée PS3, ladite activité antivirale se traduisant par l'inhibition de l'adsorption des souches virales en question sur la cellule hôte.

Pour chaque souche virale, on a mesuré la concentration efficace (CE50%), c'est-à-dire la concentration du polysaccharide sulfaté qui permet d'inhiber l'infection de 50 % des cellules d'une culture.

On a ainsi pu mettre en évidence le mécanisme d'action de la laminarine sulfatée PS3 en déterminant son effet sur l'adsorption virale. On a mis en oeuvre la laminarine sulfatée PS3 soit avant, pendant ou après l'infection des cellules Vero par différentes souches virales, soit en continu.

A titre de comparaison, on a effectué les mêmes expériences en utilisant trois produits de comparaison à savoir le sulfate de dextran, l'acyclovir et la phycarine qui est la forme non sulfatée de la laminarine extraite à l'exemple 1.

**Exemple 3 : Mesure de l'activité anti-HSV-1 de la laminarine sulfatée PS3**

Dans une première série d'expériences, on a eu recours à la méthode de viabilité cellulaire pour déterminer l'activité anti-herpétique de la souche virale HSV-1 dont le titre infectieux viral est $2x10^{6,05}$ $DI_{50}$/ml.

On a évalué cette activité anti-herpétique en déterminant les concentrations effectives ($CE_{50}$) de PS3 qui permettent de protéger 50% des cellules d'une culture contre l'infection par des suspensions virales HSV-1 ayant des MOI différentes, en fonction de la durée d'incubation du PS3 (48, 72, 96 et 120 heures).

On a utilisé l'acyclovir comme produit de comparaison.

On a déterminé les gammes de concentration de PS3 utilisées pour une MOI donnée, à savoir

- ■ la gamme de concentrations de PS3 utilisée varie de 12,5 à 1250 µg/ml, pour une suspension virale HSV-1 ayant une MOI de 1 $DI_{50}$/cellule,
- ■ la gamme de concentrations de PS3 utilisée varie de 1,25 à 125 µg/ml, pour une suspension virale HSV-1 ayant une MOI de 0,1 $DI_{50}$/cellule,
- ■ la gamme de concentrations de PS3 utilisée varie de 0,5 à 125 µg/ml, pour une suspension virale HSV-1 ayant une MOI de 0,01 $DI_{50}$/cellule.

On a réalisé la détermination des $CE_{50}$ dans des plaques à microtitration de 96 puits selon le protocole suivant. Des cellules Vero sont ensemencées à une densité cellulaire de $3{,}5.10^4$ cellules/puits dans 100 µl milieu MEM contenant 8% de SVF. Pour chaque suspension virale définie par sa MOI :

- ■ on a complété la colonne témoin négatif avec 100 µl de milieu MEM avec 8% de SVF,
- ■ on a complété la colonne témoin virus avec 50 µl de la suspension virale à la MOI voulue et avec 50 µl de milieu MEM avec 8% de SVF,
- ■ on a complété la colonne témoin PS3 ou acyclovir avec 50 µl de PS3 à la gamme de concentration voulue ou avec 50 µl d'acyclovir (Zovirax 0,05 - 5 µg/ml),
- ■ on a enfin successivement complété toutes les autres colonnes avec 50 µL de PS3 à la gamme de concentration voulue ou avec 50 µl d'acyclovir (Zovirax 0,05 - 5 µg/ml) et avec 50 µl de suspension virale à la MOI voulue.

On a ensuite incubé les cellules dans une étuve à 37°C pendant 48, 72, 96 et 120 heures et on a évalué la viabilité des cellules dans chaque puits après coloration par le rouge neutre selon la méthode décrite ci-dessus.

On a calculé la $CE_{50}$ correspondant à la concentration permettant de diminuer de 50 % l'effet cytopathique du virus selon la formule suivante :

$$\frac{DO_{(cellules+virus+PS3\ ou\ acyclovir)} - DO_{(témoin\ virus)}}{DO_{(cellules\ +\ PS3\ ou\ acyclovir)} - DO_{(témoin\ virus)}}$$

La Figure 2 résume l'activité cinétique anti-herpétique du PS3 pour les trois différentes MOI. Pour chaque temps d'incubation, c'est pour la MOI de 0,01 $DI_{50}$/cellule que l'on a enregistré les valeurs de $CE_{50}$ les plus faibles et, inversement, c'est pour la MOI de 1 $DI_{50}$/cellule que l'on a enregistré les valeurs de $CE_{50}$ les plus élevées.

**Exemple 4 : Mesure comparée de l'activité antivirale de la laminarine sulfatée PS3 sur les souches HSV-1, HSV-1R, HSV-2 et HSV-2R**

On a évalué et comparé l'activité anti-herpétique du PS3 sur chaque suspension virale HSV-1, HSV-1R, HSV-2 et HSV-2R définie par une MOI de 0,01 $DI_{50}$/cellule pour une concentration cellulaire de 350 000 cellules/ml, après des temps d'incubation de 48 et 72 heures.

On a réalisé cette série d'expériences en appliquant le protocole permettant de déterminer les $CE_{50}$, tel que décrit dans l'exemple 3. Les figures 3 et 4 permettent de comparer les $CE_{50}$ du PS3 sur les différentes souches pour des temps d'incubation de 48 et 72 heures respectivement, le produit de comparaison étant l'acyclovir.

L'examen de ces figures permet de conclure que le PS3 est plus efficace que l'acyclovir pour protéger les cellules Vero contre les souches HSV-1R, HSV-2 et HSV-2R, c'est-à-dire contre les souches virales résistantes à l'acyclovir.

En effet, la valeur $CE_{50}$ du PS3 est plus faible que celle de l'acyclovir pour chacune de ces souches, quel que soit le temps de traitement.

**Exemple 5 : Mesure de l'activité cytotoxique de la laminarine sulfatée PS3**

Pour déterminer si le PS3 a une activité cytotoxique, on a procédé de la façon suivante. Dans chaque puits, on a ensemencé les cellules Vero à une densité cellulaire de $3{,}5.10^5$ cellules/ml dans 100 $\mu$l de milieu MEM contenant 8% de SVF. On a traité les cellules par 50 $\mu$l de PS3 dont la concentration varie de 0,125 à 2500 $\mu$g/ml ou par 50 $\mu$l d'acyclovir à une concentration de 5 ; 1 ; 0,5 ; 0,1 et 0,05 $\mu$g/ml. On a ajouté 50 $\mu$l de milieu MEM contenant 8% de SVF à chaque puits. On a complété les puits contenant des cellules non traitées par 100 $\mu$l de milieu MEM contenant 8% de SVF. On a alors incubé les cellules dans une étuve à 37°C pendant 48, 72, 96 heures.

On a déterminé l'activité cytotoxique du PS3 par la mesure de la concentration cytotoxique à 50% ou $CC_{50}$. La $CC_{50}$ correspond à la concentration de PS3 qui inhibe la croissance de 50 % des cellules d'une culture. On a mesuré la valeur $CC_{50}$, exprimée en pourcentage de destruction cellulaire par la méthode de viabilité cellulaire après coloration par le rouge neutre selon la méthode décrite ci-dessus selon la formule :

$$\frac{\text{Densité optique}_{(\text{cellules non traitées})} - \text{Densité optique}_{(\text{cellules traitées})}}{\text{Densité optique}_{(\text{cellules non traitées})}} \times 100$$

La figure 5 illustre le pourcentage de destruction cellulaire ($CC_{50}$) pour la plus forte concentration de PS3, à savoir 2500 $\mu$g/ml en fonction du temps d'incubation (48, 72 et 96 heures). L'examen de la figure 5 révèle que le PS3 n'a aucun effet toxique pour les cellules Vero quel que soit le temps d'incubation.

**Exemple 6 : Mesure de l'indice thérapeutique de la laminarine sulfatée PS3, de l'acyclovir, du sulfate de dextran et de la phycarine**

Parallèlement, on a déterminé les indices thérapeutiques (IT) du PS3, ainsi que de trois autres produits de référence, à savoir l'acyclovir, le sulfate de dextran et la phycarine pour chaque souche virale. Les valeurs des IT sont résumées dans le tableau I ci dessous.

TABLEAU I

| Temps d'incubation | substances | Indice thérapeutique | | | |
|---|---|---|---|---|---|
| | | HSV-1 | HSV-1R | HSV-2 | HSV-2R |
| 48 heures | PS3 | > 1761 | > 1157 | > 4808 | > 3788 |
| | Acyclovir | > 4902 | 0 | > 579 | 0 |
| | Sulfate de dextran | - | - | - | - |
| | phycarine | - | - | - | |
| 72 heures | PS3 | > 651 | > 530 | > 1344 | > 1712 |
| | Acyclovir | > 1429 | 0 | > 613 | 0 |
| | Sulfate de dextran | > 868 | > 641 | > 3571 | > 4545 |

(suite)

| Temps d'incubation | substances | Indice thérapeutique | | | |
|---|---|---|---|---|---|
| | | HSV-1 | HSV-1R | HSV-2 | HSV-2R |
| | phycarine | 0 | 0 | 0 | 0 |
| - valeur non déterminée | | | | | |

L'indice thérapeutique (IT) représente le rapport entre la dose maximale non toxique (ou une dose induisant une toxicité quantifiée) et la dose induisant un effet pharmacologique favorable.

Dans le tableau I sont réunies les valeurs des IT du PS3, de l'acyclovir, du sulfate de dextran et de la phycarine obtenues pour chacune des quatre souches virales, après des temps d'incubation de 48 et 72 heures.

L'examen des valeurs réunies dans le tableau I permet de conclure

- ■ que le PS3 est aussi efficace que le sulfate de dextran sur toutes les souches étudiées pour des concentrations identiques (0,125 - 1250 μg/ml),
- ■ que l'acyclovir n'a aucune efficacité sur les souches HSV-1R et HSV-2R, contrairement au PS3 et
- ■ que la phycarine qui correspond à la forme non sulfatée du PS3 ne présente aucune activité anti-herpétique.

Au vu d'une part des résultats traduits par les figures 3 et 4 et d'autre part de ceux réunis au tableau I, il apparaît que le PS3 est efficace sur toutes les souches virales étudiées, avec des valeurs de $CE_{50}$ inférieures à 2 μg/ml après 48 heures de traitement et inférieures à 5 μg/ml après 72 heures de traitement.

Il est particulièrement intéressant de noter que le PS3 est efficace sur les souches résistantes à l'acyclovir qui est l'inhibiteur couramment utilisé chez les patients atteints d'herpès.

**Exemple 7 : Détermination du mécanisme d'action de la laminarine sulfatée PS3**

On a réalisé une série d'expériences afin de déterminer le mécanisme d'action du PS3, chaque étape du cycle de réplication du virus constituant une cible thérapeutique potentielle pour combattre le virus.

On a testé les susdites suspensions virales HSV-1, HSV-1R, HSV-2 et HSV-2R définies par une MOI de 0,01 $DI_{50}$/cellule pour une concentration cellulaire de 350 000 cellules/ml.

*Action du PS3 sur l'infection virale*

On a utilisé, comme produits de référence dans cette série d'expériences, l'acyclovir et le sulfate de dextran mis en oeuvre à des concentrations comprises entre 0,05 et 5 μg/ml. On a utilisé des concentrations de PS3 de 0,125 à 1250 μg/ml et on a réalisé les expériences dans des plaques à microtitration de 96 puits.

Pour vérifier si le PS3 protège une culture cellulaire lorsqu'il est mis en oeuvre avant son infection par les susdites souches virales, on a procédé de la façon suivante.

On a ensemencé des cellules Vero à une densité cellulaire de $3{,}5.10^4$ cellules/puits dans 100 μl de milieu MEM contenant 8% de SVF.

On a alors ajouté une solution de 50 μl de PS3 ou de produit de comparaison, c'est-à-dire d'acyclovir ayant une concentration donnée, à chaque puits complété de 50 μl de milieu MEM avec 8% de SVF.

On a ensuite placé la plaque de microtitration comportant les cellules traitées avec le PS3 ou le produit de référence dans une étuve à 37°C pendant 6, 12 ou 24 heures. On a ensuite lavé les cellules avec 100 μl de milieu MEM afin d'enlever le PS3 ou le produit de référence.

On a alors ajouté un volume de 50 μl d'une suspension virale dans chaque puits, ainsi que 50 μl de milieu MEM avec 8% de SVF ; et on a maintenu les plaques pendant 1 heure à 4°C.

On a ensuite placé les cellules infectées dans une étuve à 37°C pendant 48 ou 72 heures.

On a évalué la viabilité des cellules dans chaque puits par coloration par le rouge neutre selon la méthode décrite plus haut.

Au vu des résultats obtenus, on a pu conclure que les cellules prétraitées avant l'infection avec du PS3 ou de l'acyclovir n'étaient protégées contre aucune des souches étudiées.

Le PS3 ne protège donc pas les cellules de l'infection virale.

*Action virucide du PS3*

Pour déterminer si le PS3 a un effet virucide, on a procédé de la façon suivante.

On a ensemencé des cellules Vero pendant 24 heures, avant l'essai, à une densité cellulaire de $3,5.10^4$ cellules/puits d'une plaque de microtitration dans 100 μL de milieu MEM contenant 8% de SVF.

Le jour de l'essai, on a traité 50 μl de chacune des suspensions virales identifiées plus haut avec 50 μl de PS3 ou de produit de référence (acyclovir) ayant une concentration donnée pendant une heure à 37°C.

On a introduit une quantité de 100 μl du mélange de cette suspension virale à laquelle a été ajouté le PS3 ou le produit de référence dans chaque puits de la plaque de microtitration. On a maintenu pendant une heure à 4°C.

Il est à noter que l'on a réalisé des témoins de la croissance des cellules et des virus simultanément.

On a ensuite placé les plaques contenant les cellules infectées dans une étuve à 37°C pendant 48 ou 72 heures.

On a évalué l'effet virucide du PS3 par la mesure des valeurs $CE_{50}$ déterminées selon la méthode décrite à l'exemple 3.

Les figures 6 et 7 donnent les $CE_{50}$ du PS3 sur les différentes souches pour des temps d'incubation de 48 et 72 heures respectivement, le produit de comparaison étant l'acyclovir.

Si le PS3 avait une activité virucide, les valeurs $CE_{50}$ (illustrées par les figures 6 et 7) devraient être inférieures à celles obtenues lors des évaluations d'activité anti-herpétique (illustrées par les figures 3 et 4). L'examen des résultats réunis dans ces figures 6 et 7 montre que les valeurs de $CE_{50}$ sont comparables à celles obtenues lors des évaluations d'activité anti-herpétique. Le PS3 n'a donc aucun effet virucide sur les souches étudiées.

*Action du PS3 en fonction du moment de sa mise en oeuvre*

Pour déterminer à quel moment il convient de mettre le PS3 en oeuvre, on a procédé de la façon suivante.

On a ensemencé des cellules Vero à une densité cellulaire de $3,5.10^4$ cellules/puits une plaque de microtitration à 96 puits dans 100 μl de milieu MEM contenant 8% de SVF puis on les a infecté par mise en contact avec les différentes souches virales identifiées plus haut pendant 1 heure à 4°C.

On a alors lavé les cellules infectées avec un tampon salin afin d'éliminer les particules virales n'ayant pas été adsorbées.

On a ensuite traité les cellules infectées avec du PS3 ou avec le produit de comparaison :

- soit au moment de l'infection,
- soit 1, 2, 3 et 5 heures après l'infection.

Dans chaque cas on a maintenu les plaques de microtitration à 37°C pendant 72 heures. Ensuite on a déterminé l'effet inhibiteur du PS3 ou du produit de comparaison, ici l'acyclovir, pour chaque souche virale.

La figure 8 montre les valeurs de $CE_{50}$ du PS3 en fonction du moment de mise en contact du PS3 avec les cellules.

Les résultats mettent en évidence que le PS3 est particulièrement efficace sur les souches virales HSV-1R et HSV-2R même lorsqu'il est mis en oeuvre 3 heures après l'infection. L'acyclovir n'a aucun effet sur les souches virales HSV-1R et HSV-2R.

Même si l'effet inhibiteur du PS3 sur les quatre souches virales est confirmé, les résultats révèlent que la présence du PS3 au tout début de l'infection permet de conserver une efficacité à de faibles concentrations. Le PS3 est d'autant plus efficace qu'il est introduit tôt, c'est-à-dire au tout début de l'infection.

*Action du PS3 sur l'adsorption virale*

Afin de déterminer si le PS3 a un effet sur l'adsorption virale, on a procédé de la façon suivante.

On a ensemencé des cellules Vero 24 heures avant l'essai à une densité cellulaire de $3,5.10^4$ cellules/puits d'une plaque de microtitration à 96 puits dans 100 μl de milieu MEM contenant 8% de SVF. On a testé les suspensions virales HSV-1, HSV-1R, HSV-2 et HSV-2R définies par une MOI de 0,01 $DI_{50}$/cellule pour une concentration cellulaire de 350 000 cellules/ml.

Le jour de l'essai, on a soumis les cellules à trois traitements différents désignés par A, B et C :

Selon le traitement A, on a infecté les cellules traitées par 50 μl de PS3 ou de produit de comparaison (acyclovir) d'une concentration donnée avec 50 μl d'une des suspensions virales identifiées plus haut pendant une heure à 4°C.

On a ensuite lavé les cellules infectées avec 100 μl de tampon salin afin d'éliminer le PS3 ou le produit de référence ainsi que les particules virales n'ayant pas été adsorbées par les cellules.

Après avoir complété chaque puits par suffisamment de milieu MEM contenant 8% de SVF pour arriver à un volume de 200 μl, on a ensuite placé les cellules dans une étuve à 37°C pendant 72 heures.

Selon le traitement B, on a mis en contact les cellules avec l'une des suspensions virales identifiées plus haut pendant 1 heure à 4°C. On a ensuite lavé les cellules infectées avec 100 μl de tampon salin afin d'éliminer les particules virales n'ayant pas été adsorbées. On a ensuite traité les cellules pendant 72 heures avec différentes concentrations de PS3 ou du produit de référence dans un volume total de 200 μl par puits.

Selon le traitement C, on a infecté les cellules traitées par 50 μl de PS3 ou de produit de référence d'une concentration donnée avec 50 μl de l'une des suspensions virales identifiées plus haut pendant une heure à 4°C. On a ensuite lavé les cellules infectées par 100 μl de tampon salin afin d'éliminer le PS3 ou le produit de référence ainsi que les particules virales n'ayant pas été adsorbées. On a ensuite remis en contact les cellules pendant 72 heures avec les différentes concentrations de PS3 ou du produit de référence dans un volume total de 200 μl par puits.

Pour ces 3 traitements, on a évalué l'effet inhibiteur du PS3 et du produit de référence (exprimé en $CE_{50}$) sur l'adsorption des quatre susdites souches virales après 72 heures par la mesure des valeurs $CE_{50}$ selon la méthode décrite dans l'exemple 3.

La figure 9 montre les $CE_{50}$ du PS3 déterminées pour les quatre susdites souches virales identifiées plus haut après des temps d'incubation de 48 et 72 heures respectivement, le produit de comparaison étant l'acyclovir.

L'examen de cette figure 9 montre que les valeurs de $CE_{50}$ du PS3 sont très basses lors du traitement C, en particuliers pour les souches ayant développé une résistance à l'acyclovir.

En effet, on observe des valeurs de $CE_{50}$ du PS3 de 3,84 μg/ml pour la souche HSV-1, de 5,54 μg/m, pour la souche HSV-1R, de 0,24 μg/ml pour la souche HSV-2 et 0,30 μg/ml pour la souche HSV-2R.

Par conséquent, le PS3 a un effet inhibiteur sur les 4 souches virales uniquement lors du traitement C c'est-à-dire lorsque le PS3 est présent au moment et après l'adsorption virale. Le PS3 exerce donc une activité antivirale dans la première phase de l'infection virale.

*Action du PS3 sur la pénétration virale*

Pour déterminer si le PS3 a un effet sur la pénétration virale, on a procédé de la façon suivante.

On a ensemencé des cellules Vero à une densité cellulaire de $3,5.10^4$ cellules/puits d'une plaque de microtitration dans 100 μl de milieu MEM contenant 8% de SVF.

Après 24 heures, on a mis en contact les cellules confluentes avec 50 μl d'une suspension virale pendant 1 heure à 4°C.

On a ensuite incubé le tapis cellulaire avec différentes concentrations de PS3 (0,125 - 1250 μg/ml) ou de produit de référence, ici l'acyclovir (0,005 - 5 μl/ml), à 37°C afin de faciliter la pénétration des virus.

Après des temps d'incubation de 15, 30 et 60 minutes, on a lavé les cellules correspondantes avec 100 μl de PBS puis on les a traité pendant 1 minute par 0,5 mg/ml de protéinase K (Sigma) en solution dans du PBS afin d'éliminer tous les virus extracellulaires non adsorbés.

On a arrêté ce traitement par addition de 1 mM de phénylméthylsulfonyl fluoride (Sigma) en solution dans du PBS contenant 3% de SVF.

On a ensuite lavé les cellules avec 100 μl de PBS par puits et on les a remises en culture à 37°C dans 200 μl de MEM complémenté de 8% de SVF.

On a mené des témoins de la croissance des cellules et des virus simultanément. Après 48 heures d'incubation, on a mesuré l'effet du PS3 et de l'acyclovir sur la pénétration du virus en ayant recours à la méthode du rouge neutre.

Les résultats obtenus montrent que le PS3 n'a pas d'activité anti-virale au niveau de la pénétration du virus dans la cellule dans le cas des quatre souches virales.

On a réuni dans le tableau (II) les résultats obtenus dans les expériences qui viennent d'être décrites en rapport avec les quatre souches virales identifiées plus haut.

TABLEAU II

| | PS3 Souches virales étudiées : HSV-1, HSV-2, HSV-1R, HSV-2R | Acyclovir Souches non résistantes HSV-1 et HSV-2 |
|---|---|---|
| Effet avant infection | non | non |
| Effet virucide | non | non |
| Effet en rapport avec le moment d'addition | Peu efficace après l'infection | Efficace jusqu'à 5 heures après l'infection |
| Effet sur l'adsorption | **oui** | non |
| Effet sur la pénétration | non | non |

En conclusion, le PS3 présente une activité antivirale à l'égard des quatre souches virales testées, HSV-1, HSV-1R, HSV-2, HSV-2R en agissant plus particulièrement sur l'adsorption des virus sur les cellules ; il est efficace lorsqu'il est présent dès le début de l'infection.

Il est particulièrement important de souligner que le PS3 présente un effet anti-herpétique à l'égard des souches virales résistantes à l'acyclovir.

L'invention vise donc tout particulièrement l'utilisation du PS3 pour la préparation d'un médicament pour un traitement contre les souches d'herpèsvirus résistantes à l'égard des inhibiteurs du type analogue nucléosidique, et en particulier à l'égard de l'acyclovir.

Chez les individus immunocompétents, le développement d'une infection herpétique causée par des souches de virus résistant à l'acyclovir est très rare.

En revanche, dans le cas des individus immunodéficients, ce risque de développer des infections herpétiques causées par des souches résistantes aux analogues nucléosidiques est plus grand.

Les individus immunodéficients sont ceux qui sont soumis à une thérapie immunodépressive, ceux qui ont subi une transplantation d'organes, ainsi que ceux atteints du syndrome de l'immunodéficience acquise (SIDA) ou encore ceux qui présentent des grandes altérations de la peau tels que les grands brûlés.

**Exemple 8 : Mesure de la toxicité in vivo de la laminarine sulfatée PS3**

On a également recherché une éventuelle toxicité in vivo de la laminarine sulfatée PS3 utilisée conformément à l'invention.

On a réalisé cette étude sur des lapins blancs de race New Zealand et sur des rats de race Sprague Dawley.

On a soumis les lapins blancs d'une part au test d'irritation oculaire et d'autre part au test d'irritation cutanée primaire.

A l'issue du premier de ces tests on a conclu à une action légèrement irritante et dans le deuxième à une action non irritante.

On a soumis les rats d'une part à une étude de détermination de la toxicité dermique aigüe et d'autre part à une étude de détermination de la toxicité orale aigüe.

Dans le premier cas, la dose létale dermique 50 est supérieure à 2 g/kg de poids du corps ce qui permet d'affirmer que le produit n'est pas toxique.

Dans le deuxième cas, la toxicité aigüe par voie orale peut être considérée comme supérieure à 2 g/kg de poids du corps ce qui permet, à nouveau, de classer le produit comme non toxique.

**Exemple 9 : Mesure de l'activité anti-coagulatante de la laminarine sulfatée PS3**

On a montré que l'activité anticoagulante de la laminarine sulfatée PS3 est suffisamment faible comparée à celle de l'héparine pour ne pas constituer un inconvénient dans le cadre de l'utilisation conforme à l'invention et qu'elle n'est pas cytotoxique aux concentrations les plus élevées susceptibles d'être mises en oeuvre.

Pour cela, on a déterminé l'activité anticoagulante du sulfate de laminarine PS3 obtenu à l'exemple 1 en fonction de sa concentration en comparaison avec celle de l'héparine dans les tests de coagulations classiques APTT ou « Activated Partial Thromboplastin Time », de la durée de prothrombine, du test dit « HEPTEST » et de la durée de thrombine. L'APTT reflète une interaction avec le système intrinsèque de la coagulation alors que la durée de prothrombine reflète une interaction avec la coagulation extrinsèque ; le test dit « HEPTEST » est le test classique pour la mesure de l'activité inhibitrice de l'héparine à l'égard du facteur Xa et la durée de thrombine correspond à la dernière étape de la coagulation à savoir la formation de fibrines induites par la thrombine. On a pu constater qu'au contraire de celle de l'héparine, l'activité du sulfate de laminarine PS3 dans le test dit « HEPTEST » est plus de 20 fois plus faible. De même, en rapport avec la durée de prothrombine le sulfate de laminarine PS3 n'a fait preuve d'aucun effet prononcé anticoagulant, comme dans le cas de l'héparine. L'activité spécifique (IU/mg) dans l'APTT représente 30% de l'activité de l'héparine et dans le cas de la durée de thrombine 60%. Pour empêcher totalement la coagulation, on a dû appliquer dans le cas de l'APTT une concentration 4 fois plus grande et dans le cas de la durée de thrombine une concentration 20 fois plus élevée.

Dans les tests spécifiques anti-facteur Xa et anti-thrombine en utilisant des substrats chromogènes on a constaté que le sulfate de laminarine PS3 au contraire de l'héparine ne présente ni une activité significative anti-facteur Xa dépendant de l'anti-thrombine ni une activité anti-thrombine. L'effet dans le cas de la durée de thrombine peut être considéré comme étant dû à une inhibition de thrombine dépendant du cofacteur II héparine. En raison d'une part de l'activité spécifique plus faible, d'autre part du profil dépendant de la concentration et d'autre part encore d'autres recherches relatives au mécanisme d'action, il est possible de considérer que dans le cas du sulfate de laminarine PS3, le risque de saignement est sensiblement plus faible que dans le cas de l'héparine.

Il s'ensuit que les propriétés anti-herpétiques du sulfate de laminarine PS3 pourront être avantageusement

mises à profit sans crainte d'effets secondaires indésirables sur la coagulation.

Les expériences qui ont permis d'aboutir à ces conclusions ont été réalisées sous la direction du Docteur R. SHRIVASTAVA au Service de Toxicologie de l'établissement dénommé
Elevage Scientifique des Dombes (ESD) ROMANS 01400 CHATILLON SUR CHALARONNE
en respectant les lignes directrices de l'OCDE n° 404 et 405 du 24 février 1987 pour autant qu'il s'agit des études effectuées sur les lapins blancs et les lignes directrices de l'OCDE n° 401 est 402 (1987) ainsi que la directive CEE B-1 92/69 (1992) pour autant qu'il s'agit des études effectuées sur les rats Sprague Dawley.

La laminarine sulfatée testée provenait du lot PS3 8/2001 fourni par les Laboratoires Goëmar.

Les rapports correspondants sont conservés dans les archives de la Société Demanderesse.

**Exemple 10 : Composition d'une crème à base de laminarine sulfatée PS3**

On a réalisé une crème à base de laminarine sulfatée PS3 présentant la composition suivante :

| | |
|---|---|
| Eau déminéralisée | 69,7% |
| Glycérine | 5,0% |
| Laminarine sulfatée PS3 | 1,0% |
| PEG 100 stéarate | 4,0% |
| Alcool cétéarylique | 2,0% |
| Conservateur | 1,0% |
| PEG 40 stéarate | 3,0% |
| Acétate de vitamine E | 0,5% |
| C - 12-15 alkyl benzoate | 6,5% |
| Caprylic/capric triglycerides | 5,5% |
| NaOH 0,1N | 1,8% |
| | 100% |

Il est possible de prévoir 2 à 5 applications par jour.

**Exemple 11 : Composition d'une solution pour aérosol à base de laminarine sulfatée PS3**

On a réalisé une solution pour aérosol à base de laminarine sulfatée PS3 présentant la composition suivante :

| | |
|---|---|
| Laminarine sulfatée PS3 | 2,5% |
| Chlorure de sodium | 9,0% |
| Eau déminéralisée Pharmacopée Européenne | 88,5% |

Il est possible d'administrer par jour une quantité d'aérosol correspondant à une quantité de 1000 à 10 000 μg de substance active.

**Exemple 12 : Composition d'un suppositoire à base d'un sel de potassium de sulfate d'oligo β 1-3 glucan**

On a réalisé un suppositoire à base d'un sel de potassium de sulfate d'oligo

β 1-3 glucan présentant la composition suivante :

| | |
|---|---|
| Sel de potassium de sulfate d'oligo β 1-3 glucan (DP=22, Degré de sulfatation (DS) de 2,4) | 5,0% |
| Glycérines hémisynthétiques solides | 95,0% |

Il est conseillé d'en administrer 1 ou 2 par jour.

**Exemple 13 : Composition d'une solution injectable à base d'un sel de sodium de sulfate d'oligolaminaritol**

On a réalisé une solution injectable à base d'un sel de sodium de sulfate d'oligolaminaritol présentant la composition suivante :

| | |
|---|---|
| Sel de sodium de sulfate d'oligolaminaritol (DP20, DS 2,3) | 5,0% |
| Bicarbonate de sodium | 3,0% |
| Eau ppi | 92,0% |

Sur une durée de 24 heures il est possible d'administrer de 1000 à 3000 ml de la solution injectable.

**Exemple 14 : Composition d'une solution vaginale à base d'un sel de sodium de sulfate d'oligo β 1-3 glucan**

On a réalisé une solution vaginale à base d'un sel de sodium de sulfate d'oligo β 1-3 glucan, sous la forme d'un flacon unidose de 140 mL avec canule présentant la composition suivante :

| | |
|---|---|
| Sel de sodium de sulfate d'oligo β 1-3 glucan (DP23, DS 2,4) | 0,1% |
| Chlorure de sodium | 9,0% |
| Alcool éthylique à 95° | 5,0% |
| Arôme rose | 0,2% |
| Eau purifiée | 84,7% |
| Conservateurs (chlorure de benzalkonium) | 0,2% |
| Edétate de sodium | 0,3% |
| Polysorbate 20 | 0,5% |
| | 100% |

Il est possible de faire une ou deux applications par jour.

## Revendications

**1.** Polysaccharide de formule (I)

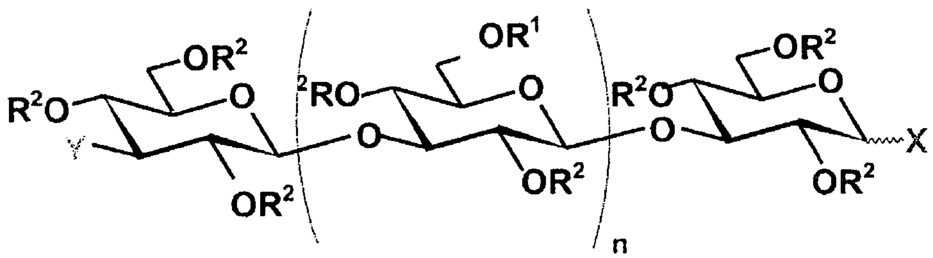

(I)

dans laquelle

- $R^1$ représente soit un atome d'hydrogène ou un groupement sulfate, soit un glucose sulfaté lié, de préférence, par une liaison de type β(1→6) à la structure saccharidique,
- $R^2$ représente un atome d'hydrogène ou un groupement sulfate, $R^1$ et $R^2$ ne pouvant pas représenter simultanément un atome d'hydrogène,
- X et Y représentent, chacun indépendamment, un groupement OH, un glucose, un glucose sulfaté, un mannitol ou un mannitol sulfaté,
- n représente un nombre entier de 11 à 30, préférentiellement de 20 à 30, plus préférentiellement de 25 à 30,

ledit polysaccharide ayant un degré de sulfatation supérieur à 2, préférentiellement de 2,2 à 2,4, pour une utilisation dans une méthode de traitement d'une maladie causée par un herpèsvirus.

**2.** Polysaccharide pour utilisation selon la revendication 1, ledit polysaccharide étant une laminarine sulfatée ayant

un degré de sulfatation supérieur à 2 et de préférence de 2,2 à 2,4.

**3.** Polysaccharide pour une utilisation dans une méthode de traitement d'une maladie causée par un herpèsvirus, ledit polysaccharide étant une laminarine sulfatée ayant un degré de sulfatation supérieur à 2 et de préférence de 2,2 à 2,4.

**4.** Polysaccharide pour utilisation selon la revendication 2 ou la revendication 3, ladite laminarine sulfatée ayant un degré de polymérisation de 11 à 28.

**5.** Polysaccharide pour utilisation selon l'une quelconque des revendications 1-4, ledit herpèsvirus étant un alpha herpèsvirus.

**6.** Polysaccharide pour utilisation on la revendication 5, ledit alpha herpèsvirus étant HSV-1 ou HSV-2

**7.** Polysaccharide pour utilisation selon l'une des revendications 1-6, ledit herpèsvirus étant résistant aux agents antiviraux déjà connus.

**8.** Polysaccharide pour utilisation selon l'une des revendications 1-7, ladite maladie causée par un herpèsvirus étant choisie parmi une maladie causée par un herpèsvirus chez un individu immunodéficient, plus particulièrement une maladie causée par un herpèsvirus chez un individu transplanté, une maladie causée par un herpèsvirus chez un individu atteint du syndrome de l'immunodéfience acquise ou une maladie causée par un herpèsvirus chez un individu qui présente de grandes altérations de la peau, et notamment une maladie causée par un herpèsvirus chez un grand brûlé.

**9.** Polysaccharide pour utilisation selon l'une des revendications 1-8, ladite méthode de traitement d'une maladie causée par un herpèsvirus comprenant en outre l'administration simultanée ou séquentielle d'une quantité efficace d'au moins un composé choisi dans le groupe constitué des inhibiteurs nucléosidiques et des inhibiteurs non-nucléosidiques d'une enzyme virale d'un herpèsvirus.

**10.** Composition pharmaceutique comprenant au titre de substance active une quantité efficace d'au moins un polysaccharide de formule (I) :

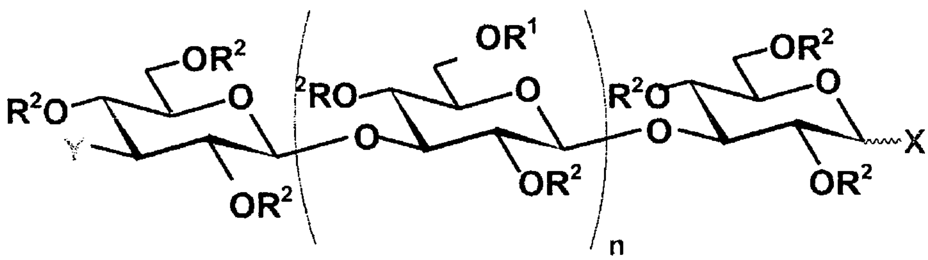

**(I)**

dans laquelle

- $R^1$ représente soit un atome d'hydrogène ou un groupement sulfate, soit un glucose sulfaté lié, de préférence, par une liaison de type $\beta(1\rightarrow6)$ à la structure saccharidique,
- $R^2$ représente un atome d'hydrogène ou un groupement sulfate, $R^1$ et $R^2$ ne pouvant pas représenter simultanément un atome d'hydrogène,
- X et Y représentent, chacun indépendamment, un groupement OH, un glucose, un glucose sulfaté, un mannitol ou un mannitol sulfaté,
- n représente un nombre entier de 11 à 30, préférentiellement de 20 à 30, plus préférentiellement de 25 à 30,

ledit polysaccharide ayant un degré de sulfatation supérieur à 2, préférentiellement de 2,2 à 2,4,
ainsi qu'une quantité efficace d'au moins un composé choisi dans le groupe constitué des inhibiteurs nucléosidiques et des inhibiteurs non-nucléosidiques d'une enzyme virale d'un herpèsvirus,
pour une utilisation simultanée, séparée ou étalée dans le temps.

**11.** Composition selon la revendication 10, ledit polysaccharide étant une laminarine sulfatée ayant un degré de sulfatation supérieur à 2 et de préférence de 2,2 à 2,4.

## Claims

**1.** Polysaccharide of formula (I)

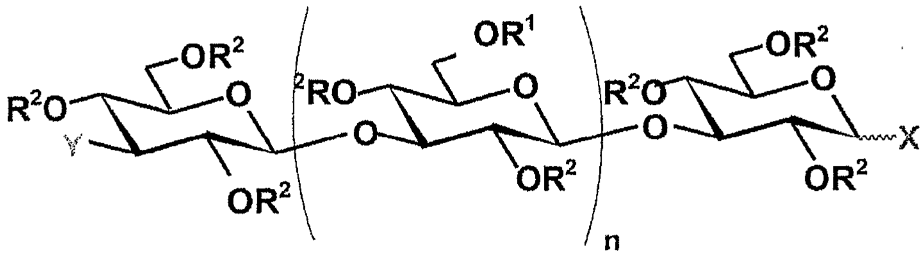

(I)

wherein

- $R^1$ represents either a hydrogen atom or a sulphate group or a sulphated glucose bonded to the saccharide structure, preferably by a bond of the β(1→6) type,
- $R^2$ represents a hydrogen atom or a sulphate group, $R^1$ and $R^2$ not being able to represent a hydrogen atom simultaneously,
- X and Y each independently represent an OH group, a glucose, a sulphated glucose, a mannitol or a sulphated mannitol,
- n represents an integer from 11 to 30, preferably from 20 to 30, more preferably from 25 to 30,

said polysaccharide having a degree of sulphation greater than 2, preferably from 2.2 to 2.4, for a use in a method for treatment of disease caused by herpes virus.

**2.** Polysaccharide for a use according to claim 1, said polysaccharide being a sulphated laminarin having a degree of sulphation greater than 2, and preferably from 2.2 to 2.4.

**3.** Polysaccharide for a use in a method for treatment of disease caused by herpes viruses, said polysaccharide being a sulphated laminarin having a degree of sulphation greater than 2, and preferably from 2.2 to 2.4.

**4.** Polysaccharide for a use according to claim 2 or claim 3, said sulphated laminarin having a degree of polymerization of 11 to 28.

**5.** Polysaccharide for a use according to any one of claims 1-4, said herpes virus being an alpha-herpes virus.

**6.** Polysaccharide for a use according to claim 5, said alpha-herpes virus being HSV-1 or HSV-2.

**7.** Polysaccharide for a use according to any one of claims 1-6, said herpes virus being resistant to known antiviral agents.

**8.** Polysaccharide for a use according to any one of claims 1-7, said disease caused by herpes viruses being chosen from a disease caused by herpes virus in immunodeficient individuals, more particularly a disease caused by a herpes virus in transplanted individual, a disease caused by a herpes virus in individual suffering from acquired immunodeficiency syndrome or a disease caused by herpes virus in individual who has major changes to the skin, and in particular a disease caused by a herpes virus in major bum.

**9.** Polysaccharide for a use according to any one of claims 1-8, said method for treatment of a disease caused by herpes virus also comprising the simultaneous or sequential administration of an effective amount of at least one compound chosen from the group consisting of nucleoside inhibitors and non-nucleoside inhibitors of a viral enzyme

of a herpes virus.

**10.** Pharmaceutical composition comprising as active agent an effective amount of at least one polysaccharide of formula (I)

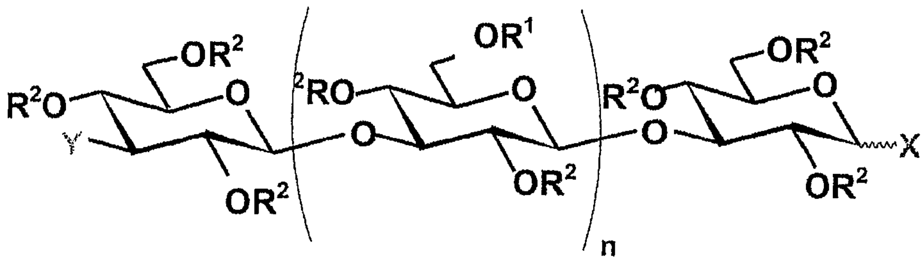

(I)

wherein

- $R^1$ represents either a hydrogen atom or a sulphate group or a sulphated glucose bonded to the saccharide structure, preferably by a bond of the β(1→6) type,
- $R^2$ represents a hydrogen atom or a sulphate group, $R^1$ and $R^2$ not being able to represent a hydrogen atom simultaneously,
- X and Y each independently represent an OH group, a glucose, a sulphated glucose, a mannitol or a sulphated mannitol,
- n represents an integer from 11 to 30, preferably from 20 to 30, more preferably from 25 to 30,

said polysaccharide having a degree of sulphation greater than 2, preferably from 2.2 to 2.4, and an effective amount of at least one compound chosen from the group consisting of nucleoside inhibitors and non-nucleoside inhibitors of a viral enzyme of a herpes virus, for a use which is simultaneous, separate or spread over time.

**11.** Composition according to claim 10, said polysaccharide being a sulphated laminarin with a degree of sulphation greater than 2 and preferably from 2.2 to 2.4

**Patentansprüche**

**1.** Polysacchrid mit der Formel (I)

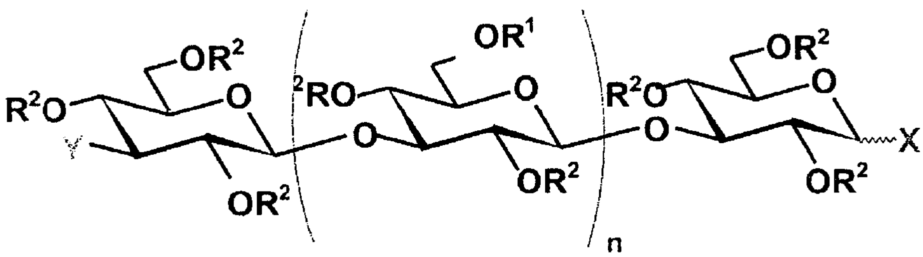

(I)

wobei

- $R^1$ entweder ein Wasserstoffatom oder eine Sulfatgruppe, oder eine sulfatierte Glucose, die vorzugsweise durch eine Bindung vom Typ β(1→6) an die Saccharidstruktur gebunden ist, ist
- $R^2$ ein Wasserstoffatom oder eine Sulfatgruppe ist, wobei $R^1$ und $R^2$ nicht gleichzeitig ein Wasserstoff sein können,
- X und Y jeweils unabhängig voneinander eine OH-Gruppe, eine Glukose, eine sulfatierte Glukose, ein Mannitol

oder ein sulfatiertes Mannitol sind
- n eine ganze Zahl zwischen 11 und 30 ist, vorzugsweise zwischen 20 und 30, besonders bevorzugt zwischen 25 und 30,

wobei das Polysaccharid einen Sulfatationsgrad größer als 2 hat, vorzugsweise zwischen 2,2 und 2,4,
zur Verwendung für eine Behandlungsmethode einer Krankheit, die durch einen Herpesvirus verursacht wird.

2. Polysaccharid zur Verwendung nach Anspruch 1, wobei das Polysaccharid ein sulfatiertes Laminarin ist, das einen Sulfatationsgrad hat, der größer als 2 ist, vorzugsweise zwischen 2,2 und 2,4.

3. Polysaccharid zur Verwendung für eine Behandlungsmethode einer Krankheit, die durch einen Herpesvirus verursacht wird,
wobei das Polysaccharid ein sulfatiertes Laminarin ist, das einen Sulfatationsgrad hat, der größer als 2 ist, vorzugsweise zwischen 2,2 und 2,4.

4. Polysaccharid zur Verwendung nach Anspruch 2 oder 3, wobei das sulfatierte Laminarin einen Polymerisationsgrad von 11 bis 28 hat.

5. Polysaccharid zur Verwendung nach einem beliebigen der Ansprüche 1 bis 4, wobei der Herpesvirus ein alpha Herpesvirus ist.

6. Polysaccharid zur Verwendung nach Anspruch 5, wobei der alpha Herpesvirus ein HSV-1 oder HSV-2 ist.

7. Polysaccharid zur Verwendung einem Ansprüche 1 bis 6, wobei der Herpesvirus gegen bereits bekannte antivirale Wirkstoffe resistent ist.

8. Polysaccharid zur Verwendung nach einem der Ansprüche 1 bis 7, wobei die Krankheit, die durch einen Herpesvirus verursacht wird, ausgewählt wird aus einer Krankheit, die durch einen Herpesvirus bei einem immundefizienten Wesen verursacht wird, insbesondere einer Krankheit, die durch einen Herpesvirus bei einem transplantierten Wesen verursacht wird, einer Krankheit, die durch einen Herpesvirus bei einem Wesen, das das Immundefizienz-Syndrom hat, verursacht wird, oder einer Krankheit, die durch einen Herpesvirus bei einem Wesen, das große Veränderungen der Haut aufweist, verursacht wird, und insbesondere einer Krankheit, die durch einen Herpesvirus bei schwerer Verbrennung verursacht wird.

9. Polysaccharid zur Verwendung nach einem der Ansprüche 1 bis 8, wobei die Behandlungsmethode einer Krankheit, die durch einen Herpesvirus verursacht wird, unter anderem die gleichzeitige oder sequentielle Verabreichung einer effizienten Menge mindestens eines Stoffes aufweist, der aus der Gruppe, die Nukleosidinhibitoren und Nichtnukleosidinhibitoren eines viralen Enzyms eines Herpesvirus umfasst, ausgewählt wird.

10. Pharmazeutische Zusammensetzung, die als Wirkstoff eine effiziente Menge mindestens eines Polysaccharids mit der Formel (I) umfasst:

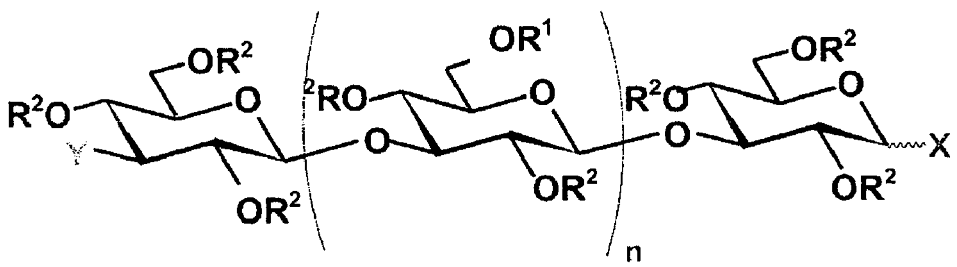

(I)

wobei

- $R^1$ entweder ein Wasserstoffatom oder eine Sulfatgruppe, oder eine sulfatierte Glukose, die vorzugsweise durch eine Bindung vom Typ $\beta(1\rightarrow6)$ an die Saccharidstruktur gebunden ist, ist

- $R^2$ ein Wasserstoffatom oder eine Sulfatgruppe ist, wobei $R^1$ und $R^2$ nicht gleichzeitig ein Wasserstoffatom sein können,
- X und Y jeweils unabhängig voneinander eine OH-Gruppe, eine Glukose, eine sulfatierte Glukose, ein Mannitol oder ein sulfatiertes Mannitol sind,
- n eine ganze Zahl zwischen 11 und 30 ist, vorzugsweise zwischen 20 und 30, besonders bevorzugt zwischen 25 und 30,

wobei das Polysaccharid einen Sulfatationsgrad hat, der größer als 2 ist, vorzugsweise zwischen 2,2 und 2,4, sowie eine effiziente Menge mindestens eines Stoffes, der aus der Gruppe, die Nukleosidinhibitoren und Nichtnukleosidinhibitoren eines viralen Enzyms eines Herpesvirus umfasst, ausgewählt wird, zur gleichzeitigen, separaten oder zeitlich verteilten Anwendung.

**11.** Zusammensetzung nach Anspruch 10, wobei das Polysaccharid ein sulfatiertes Laminarin ist, das einen Sulfatationsgrad hat, der größer als 2 ist, vorzugsweise zwischen 2,2 und 2,4.

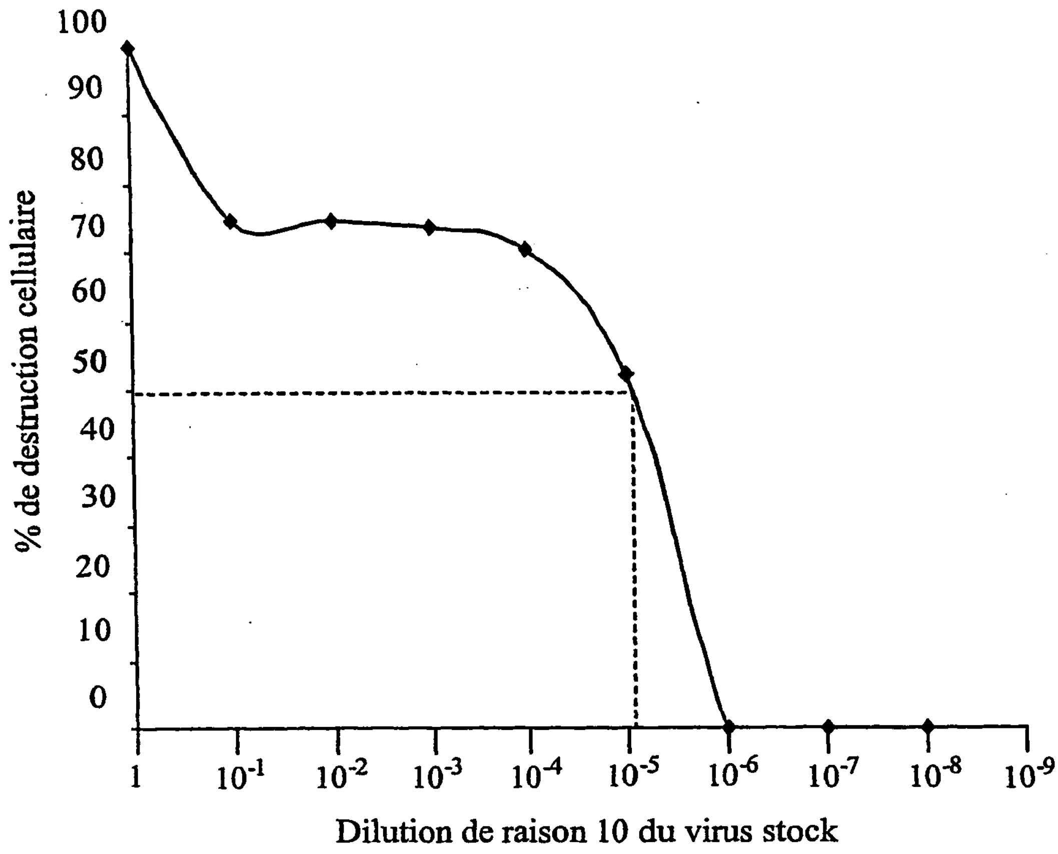
100
90
80
70
60
50
40
30
20
10
0
% de destruction cellulaire
1
10-1
10-2
10-3
10-4
10-5
10-6
10-7
10-8
10-9
Dilution de raison 10 du virus stock

FIG. 1

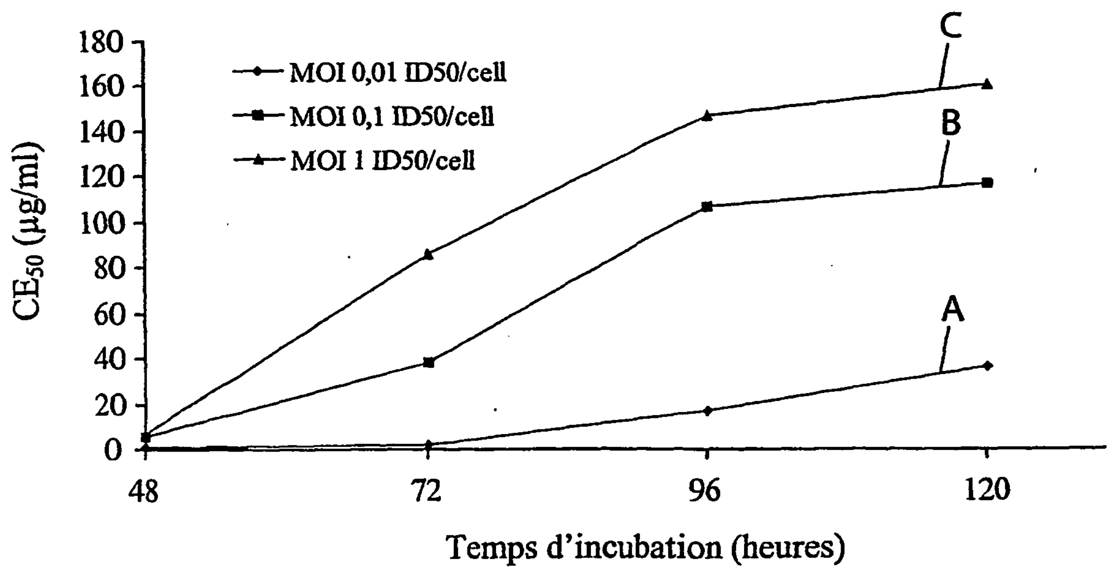
180
160
140
120
100
80
60
40
20
0
CE50 (µg/ml)
MOI 0,01 ID50/cell
MOI 0,1 ID50/cell
MOI 1 ID50/cell
C
B
A
48
72
96
120
Temps d'incubation (heures)

FIG. 2

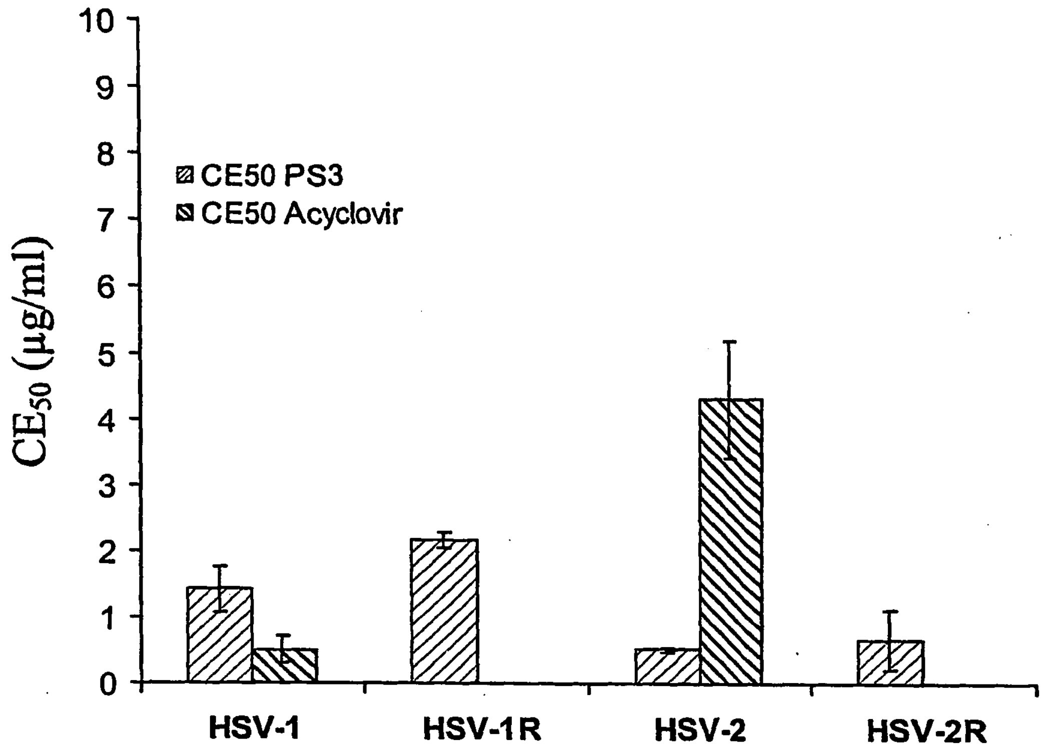
10
9
8
7
6
5
4
3
2
1
0
CE50 PS3
CE50 Acyclovir
CE$_{50}$ (µg/ml)
HSV-1
HSV-1R
HSV-2
HSV-2R

FIG. 3

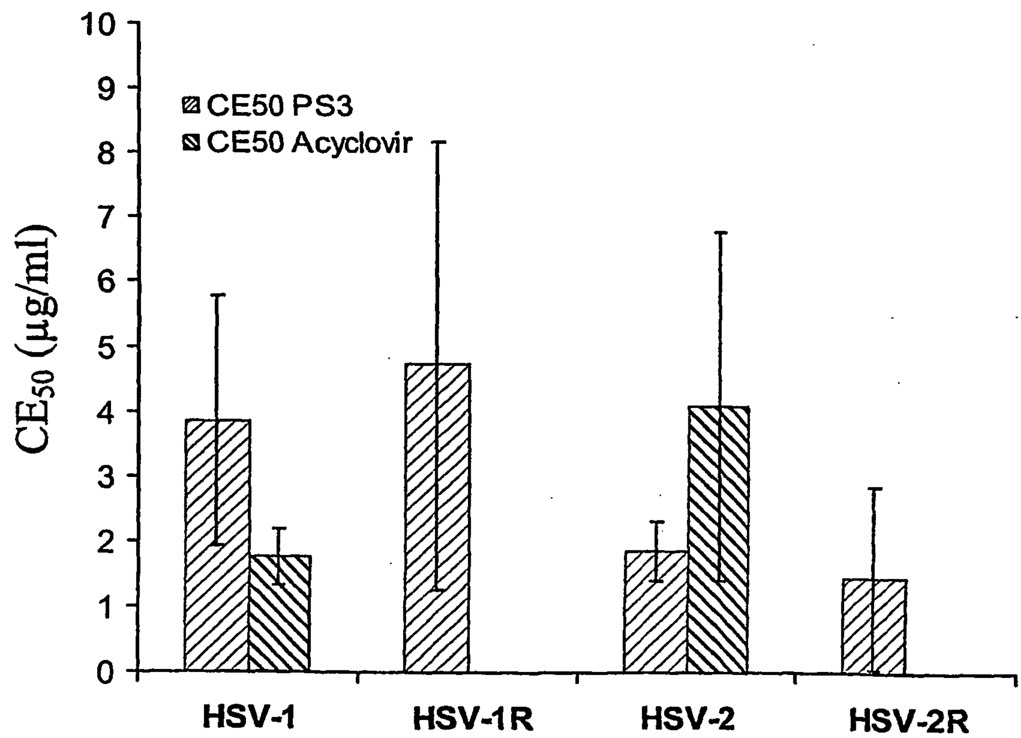
10
9
8
7
6
5
4
3
2
1
0
CE50 PS3
CE50 Acyclovir
CE$_{50}$ (µg/ml)
HSV-1
HSV-1R
HSV-2
HSV-2R

FIG. 4

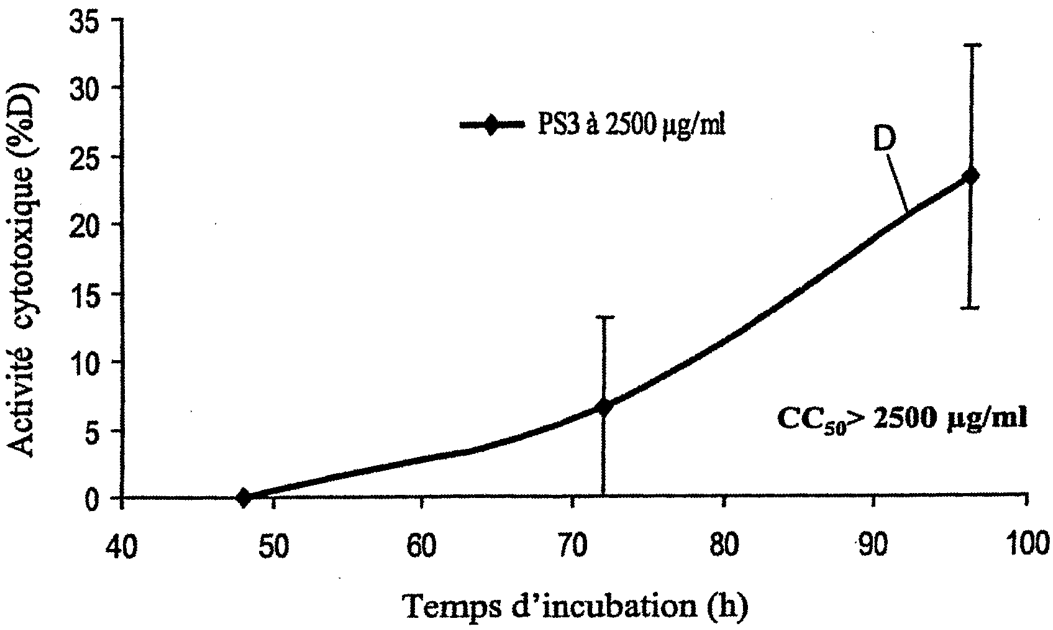
35
30
25
20
15
10
5
0
Activité cytotoxique (%D)
PS3 à 2500 µg/ml
D
CC50> 2500 µg/ml
40
50
60
70
80
90
100
Temps d'incubation (h)

FIG. 5

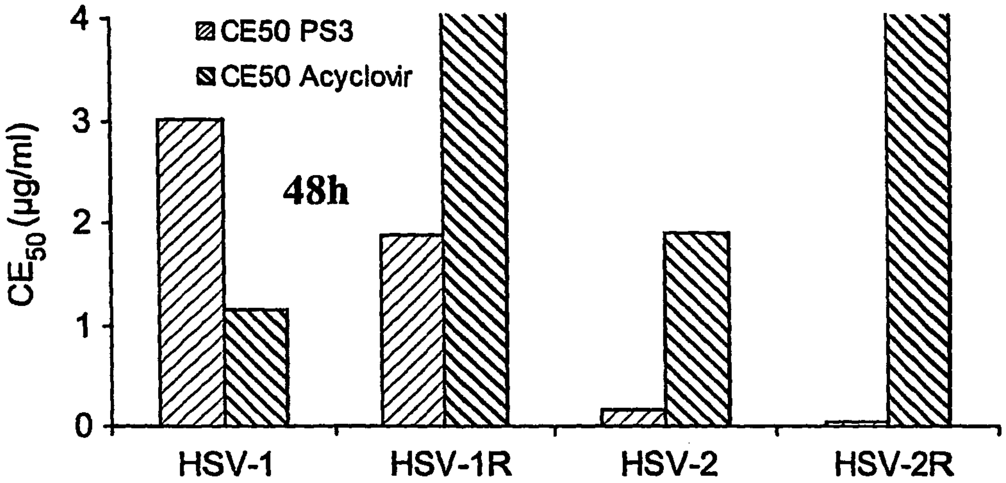
CE50 PS3
CE50 Acyclovir
48h
$CE_{50}$ (µg/ml)
0
1
2
3
4
HSV-1
HSV-1R
HSV-2
HSV-2R

FIG. 6

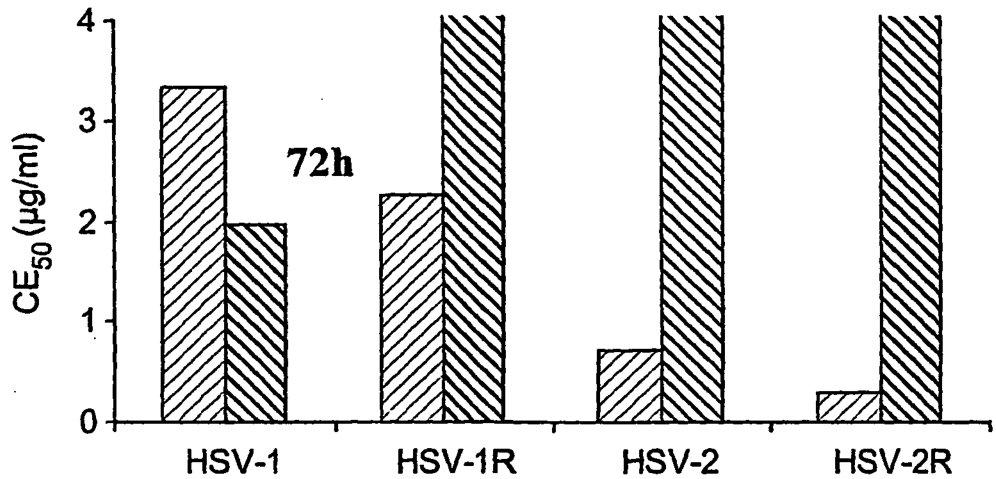
72h
$CE_{50}$ (µg/ml)
0
1
2
3
4
HSV-1
HSV-1R
HSV-2
HSV-2R

FIG. 7

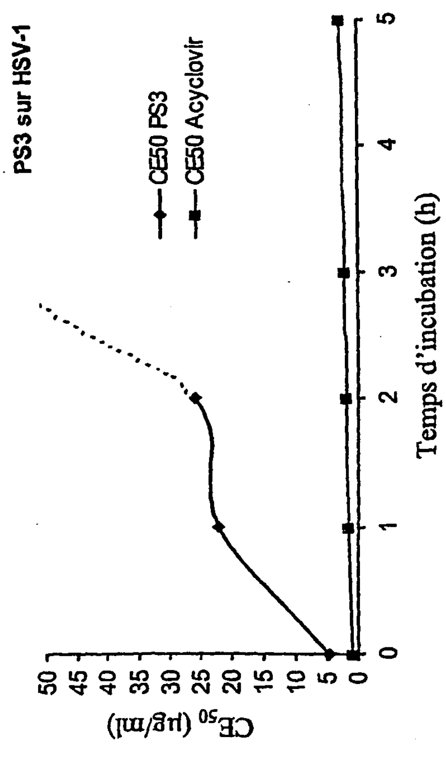


FIG. 8A

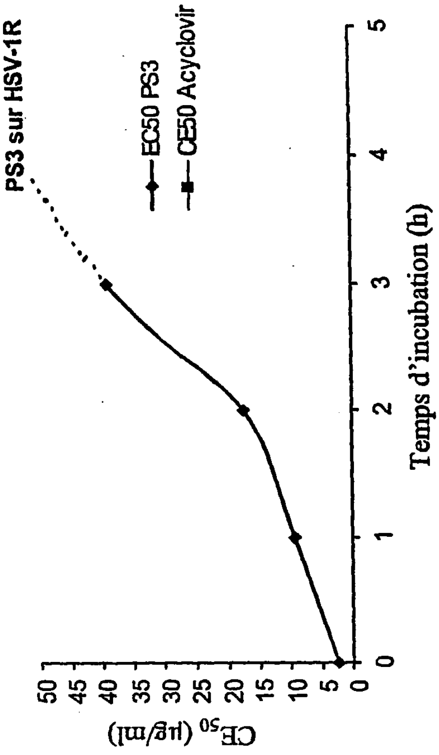


FIG. 8B

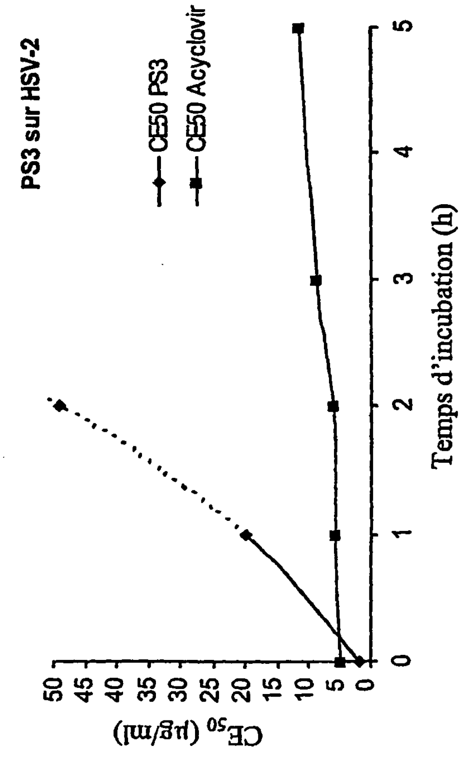


FIG. 8C

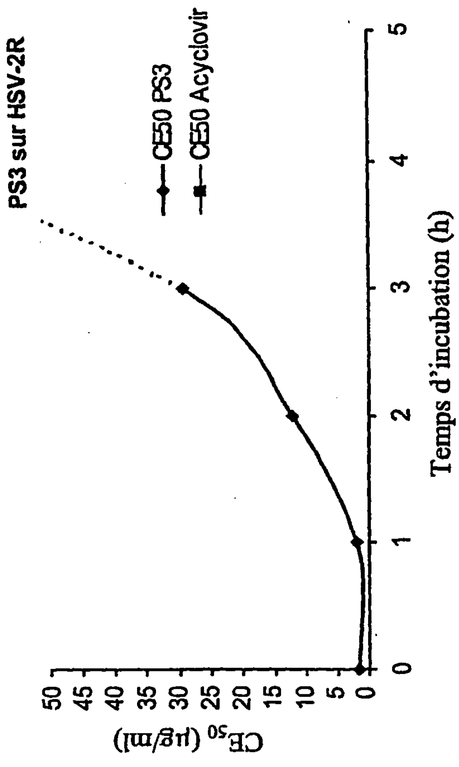


FIG. 8D

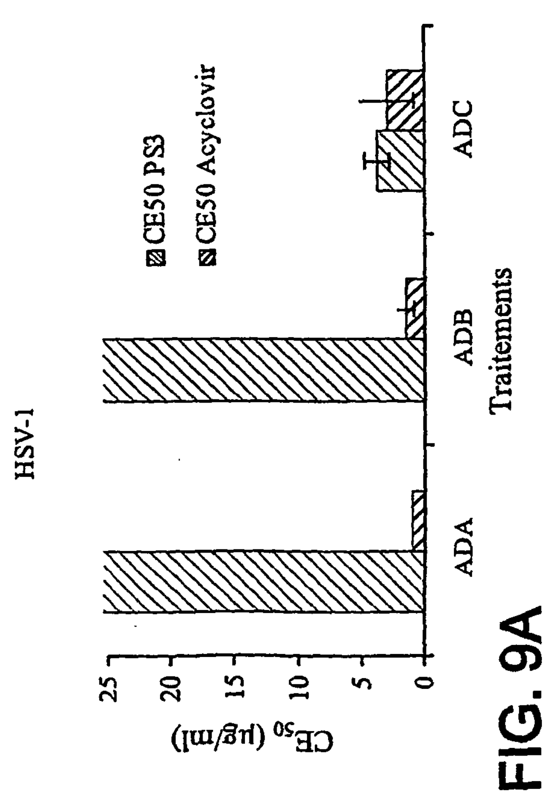


FIG. 9A

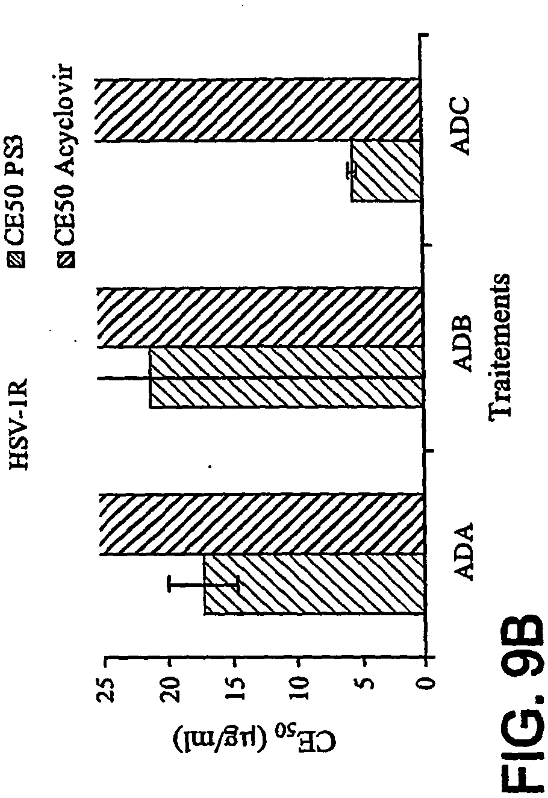


FIG. 9B

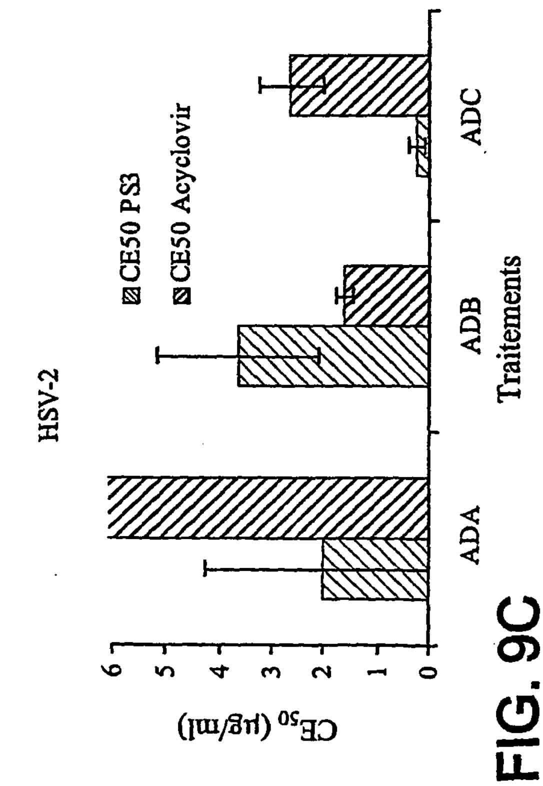


FIG. 9C

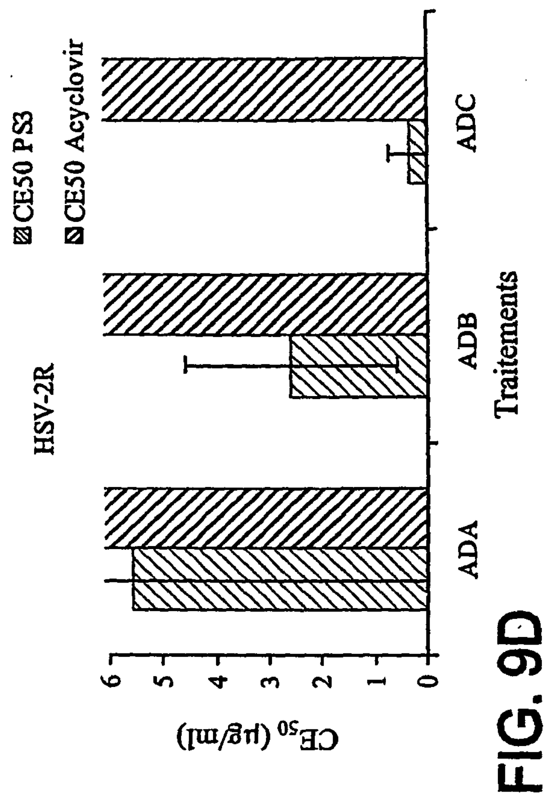


FIG. 9D

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 9208387


### Littérature non-brevet citée dans la description


- **Englund et al.** *Ann. Intern. Med.,* 1990, vol. 112, 416-22
- **Baba et al.** *Antimicrobial Agents and Chemotherapy,* 1988, vol. 32, 1742-1745
- **Mazumder et al.** *Inter. J. Biol. Macromol,* 2002, vol. 31, 87-95
- **Yingzhou et al.** *China J.I.,* 2004, vol. 6, 23
- **BABA M et al.** SULFATED POLYSACCHARIDES ARE POTENT AND SELECTIVE INHIBITORS OF VARIOUS ENVELOPED VIRUSES INCLUDING HERPES SIMPLEX VIRUS CYTOMEGALOVIRUS VESICULAR STOMATITIS VIRUS AND HUMAN IMMUNODEFICIENCY VIRUS. *ANTIMICROBIAL AGENTS AND CHEMOTHERAPY,* 1988, vol. 32 (11), 1742-1745
- **Alban S ; Kraus J ; Franz G.** Synthesis of laminarin sulfates with anticoagulant activity. *Artzneim.Forsch./drug Res,* 1992, vol. 42, 1005-1008
- **Susanne Alban.** Synthese und physiologische Testung neuartiger Heparinoide. 1993
- **Reed ; Muench.** *Am, J. Hyg.,* 1983, vol. 27, 493-497
- **McLaren et al.** *Antiviral Research,* 1983, vol. 3, 223-234
- **Langlois et al.** *Standardization,* 1986, vol. 14, 201-211